# EUROPEAN PATENT APPLICATION

(11) **EP 0 984 012 A2**
(43) Date of publication of application: **08.03.2000**
(21) Application number: 99306575.4
(22) Date of filing: 19.08.1999
(51) Int. Cl.: C07D 409/06, C07D 405/06, C07D 209/34, A61K 31/40, A61P 29/00

(54) **Nitric oxide releasing oxindole prodrugs with analgesic and anti-inflammatory properties**

(30) Priority: 31.08.1998 US 98454 P
(71) Applicant: Pfizer Products Inc., Groton, Connecticut 06340 (US)
(72) Inventor: Lundy, Kristin Marie, Groton, Connecticut 06340 (US); Clark, Michael Thomas, Gales Ferry, Connecticut 06335 (US)
(74) Representative: Simpson, Alison Elizabeth Fraser

(57) **Abstract**

Nitric oxide releasing oxindole prodrugs are described which are useful in methods of treating or preventing pain, inflammation, fever, or gastrointestinal lesions in a patient in need of such treatment, or of modifying an inflammatory disease or condition by favorably affecting the outcome thereof in said patient, wherein there is administered to said patient a therapeutically effective amount of a compound of Formula (I); 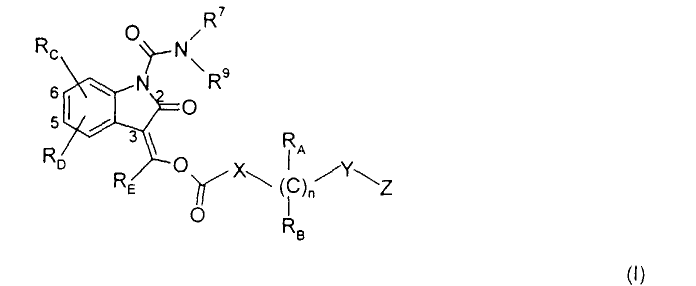 and pharmaceutically acceptable salts thereof: wherein
X is a covalent bond, -O-, -S-, or -N(R¹)-;
R_{A} and R_{B} are members independently selected from the group consisting essentially of H; (C₁-C₄)alkyl; (C₃-C₆)cycloalkyl; phenyl; or taken together with the carbon atom to which they are attached, form a bridging (C₃-C₆)cycloalkyl moiety;
n is an integer selected from 1 through 6, inclusive;
Y is a covalent bond -O-, -S-, or -N-;
Z is -NO or -NO₂;
R_{C} is a member independently selected from the group consisting essentially of H, F, Cl, Br, (C₁-C₄)alkyl, (C₃-C₇)cycloalkyl, (C₁-C₄)alkoxy, (C₁-C₄)alkylthio, CF₃, (C₁-C₄)alkylsulfinyl, (C₁-C₄)alkylsulfonyl, NO₂, phenyl, (C₂-C₄)alkanoyl, benzoyl, thenoyl, (C₂-C₄)alkanamido, benzamido, and N,N-di-(C₁-C₃)alkylsulfamoyl;
R_{D} is a member independently selected from the group consisting essentially of H, F, Cl, Br, (C₁-C₄)alkyl, (C₃-C₇)cycloalkyl, (C₁-C₄)alkoxy, (C₁-C₄)alkylthio, and CF₃; or
R_{C} and R_{D} when taken together are a methylenedioxy group or an ethylenedioxy group; or form a divalent radical D, wherein D is selected from the group consisting essentially of 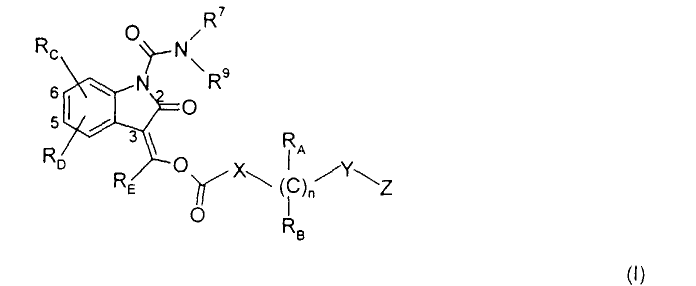 wherein W is O or S;
R_{E} is a member independently selected from the group consisting essentially of (C₁-C₆)alkyl, (C₃-C₇)cycloalkyl, (C₄-C₇)cycloalkenyl, phenyl, phenyl(C₁-C₃)alkyl, phenoxy(C₁-C₃)alkyl, thiophenoxy(C₁-C₃)alkyl, naphtyl, bicyclo[2.2.1]heptan-2-yl, bicyclo[2.2.1]hept-5-en-2-yl, and -(CH₂)ₘ-Q-R²;
R⁷ and R⁹ are independently selected from the group consisting essentially of hydrogen, (C₁-C₃)alkyl, -C(=O)(C₁-C₃)alkyl, phenyl, and -C(=O)N(R⁸)(R¹⁰).

## Description

### BACKGROUND OF THE INVENTION

The present invention relates to novel compositions of matter which are non-steroidal anti-inflammatory drugs (NSAIDs) chemically linked to a nitric oxide (NO) releasing moiety, in order to eliminate or reduce undesired gastrointestinal side effects which would otherwise be produced by the NSAID acting alone. This improvement in the therapeutic index of said NSAIDs is based on the rationale that since NO maintains gastric mucosal blood flow and prevents leukocyte adherence within the gastric microcirculation, that NO will be capable of counteracting the detrimental effects of cyclooxygenase suppression caused by the NSAID itself.

### DESCRIPTION OF THE STATE OF THE ART

For a considerable number of years conditions in humans and other mammals involving inflammation, fever, pain and thrombosis have been treated with non-steroidal anti-inflammatory drugs, known as NSAIDs, and currently these drugs collectively are among the most widely prescribed in the world. The considerable majority of NSAIDs in use today achieve their anti-inflammatory, anti-pyretic, analgesic and anti-thrombotic effects by way of a mechanism of action that inhibits the action of the prostaglandin G/H synthase enzyme, *i.e.*, cyclooxygenase, on its substrate arachidonic acid. Unfortunately, this also results in significant gastric toxicity and unwanted effects on the gastrointestinal tract. NSAIDs disrupt the arachidonic acid cascade, which normally leads to the endogenous production of prostaglandins, thromboxanes and leukotrienes which are mediators of inflammation, but also to the endogenous production of certain prostaglandins which have a protective action with regard to the mucosal lining of the stomach and other portions of the gastrointestinal tract.

Initially, a number of different strategies were adopted in the art in order to prevent or at least ameliorate the gastrointestinal injury caused by most NSAIDs. These approaches included enteric coating of NSAIDs to prevent absorption in the stomach and to block the topical irritant property of some NSAIDs; parenteral administration to temporarily bypass the gastrointestinal system; the formulation of prodrug forms which require hepatic metabolism in order for the active form to be produced; and the co-administration of acid secretion inhibitors such as H₂-receptor antagonists, or of exogenous prostaglandins in order to reestablish their presence in the mucosa of the gastrointestinal tract. Despite the plausibility and varying character of the above-discussed approaches, none proved able to favorably impact the incidence of severe adverse reactions to NSAIDs, such as gastrointestinal perforation and bleeding.

More recently, there emerged in the art two new approaches to overcoming the toxicity of NSAIDs which differed significantly from the above-discussed approaches One of these was based on the discovery that there is a second form of the originally recognized cyclooxygenase, which is now referred to as the constitutively expressed isoform of cyclooxygenase, COX-1. The second, inducible isoform of the enzyme is now referred to as COX-2, and it can be induced by interleukin 1 (IL-1) and endotoxin, and its expression is upregulated at sites of inflammation. COX-2 is found in fibroblasts, endothelial cells, macrophages, chondrocytes and mesangial cells. The discovery of the existence of inducible COX-2 led to a search for selective inhibitors which would be capable of suppressing prostaglandin production at sites of inflammation induced by endotoxin or IL-1, while at the same time having little or no impact on the production of prostaglandins in other tissues, especially the gastrointestinal tract.

While the development of selective COX-2 inhibitors appeared to be an eminently rational approach in the art, some challenges to the success of that approach were pointed out by those skilled in the art. These challenges included the possibility that prostanoids produced by COX-1 also played a contributory role in causing inflammation, pain and fever. Since many existing NSAIDs substantially inhibited both COX-1 and COX-2, it was possible that a selective COX-2 inhibitor might be less therapeutically effective than many then existing NSAIDs. Other challenges included the possibility that prostanoids produced by COX-2 were physiologically important; the possibility that selective COX-2 inhibitors would not be effective in the small intestine since injury produced by NSAIDs in the small intestine, unlike the injury produced in the stomach, was not related to the suppression of prostaglandin synthesis; and the possibility that selective COX-2 inhibitors would not be useful as anti-thrombotic agents, since thromboxane synthesis by platelets was known to be mediated by COX-1.

Since the art was aware of the above-discussed potential drawbacks to the usefulness of selective COX-2 inhibitors, there emerged in the art at about the same time a second, different approach to reducing the gastrointestinal toxicity of NSAIDs. This second approach involved the chemical linking of a nitric oxide (NO) releasing moiety to the structure of an existing NSAID, based on the rationale that since NO maintained gastric mucosal blood flow and prevented leukocyte adherence within the gastric microcirculation, that NO might be capable of counteracting the detrimental effects of cyclooxygenase suppression. The validity of this approach was established when it was shown that flurbiprofen nitroxybutylester, ketoprofen nitroxybutylester, and diclofenac nitroxybutylester all possessed anti-inflammatory activity comparable to that of their parent NSAIDs in accepted animal models of acute and chronic inflammation, while at the same time these NO-releasing compounds resulted in markedly less gastric injury than that produced by their parent NSAIDs, when administered on both an acute and a chronic basis.

It was also found that the NO-releasing NSAID derivatives caused no detectable injury to the small intestines of test animals, while the parent NSAIDs caused significant injury following repeated administration, which eventually led to perforation of the small intestine and death of the test animal. Further still, it was discovered that NO-releasing NSAID derivatives had enhanced anti-platelet activity, which apparently was attributable to the ability of NO to inhibit platelet adhesion and aggregation. This discovery appeared to indicate that NO-releasing NSAID derivatives would also be useful as anti-thrombotic agents. Accordingly, it was established in the art already that the development of NO-releasing NSAID derivatives represented a valid approach for the discovery and development of anti-inflammatory agents which do not possess gastrointestinal toxicity. These developments in the art are detailed in Wallace, J. L. and Cirino, G., "The Development of Gastrointestinal-sparing Nonsteroidal Anti-inflammatory Drugs", *TiPS,* 15, 1994, 405-406.

The ulcerogenic properties of existing NSAIDs was well known in the art, as above-described. It was also known that existing NSAIDs interfered with the healing of ulcers, thus further limiting their usefulness. Accordingly, the effect of NO-releasing NSAID derivatives on the healing of experimental ulcers was also investigated in the art, since NO was expected to inhibit the release of reactive oxygen metabolites and proteases from white blood cells within the gastrointestinal microcirculation, and since a reduction in such circulation as well as leukocyte stimulation had been implicated in the pathogenesis of NSAID gastropathy. It was also known that the NO donor, glyceryl trinitrate, had significantly accelerated the healing of experimental gastric ulcers in the rat. The effect on healing of experimental gastric ulcers in the rat by daily treatment with the NO-releasing NSAID nitrofenac, diclofenac-4-nitroxybutylester, compared to the activity of diclofenac, the NSAID parent compound of nitrofenac, nabumetone, a COX-2 selective NSAID, and the NO donor glyceryl trinitrate, was studied by Elliott, S. N.; McKnight, W.; Cirino, G.; and Wallace, J. L.; "A Nitric Oxide-Releasing Nonsteroidal Anti-inflammatory Drug Accelerates Gastric Ulcer Healing in Rats", *Gastroenterology,* 109, 1995, 524-530. It was concluded from the study that healing of the experimental gastric ulcers was accelerated by nitrofenac and glyceryl trinitrate, and that diclofenac and nabumetone had no effect on the healing rates.

Safety issues were investigated in the art after reduction in gastric and small intestinal injury was observed with the NO-releasing NSAID derivatives. The NO-releasing NSAID derivatives were noted to be esters of nitric acid and consequently close chemical relatives of nitroglycerin, which achieves its cardiovascular effects by releasing NO within the vascular space. Although an adequate anti-inflammatory dose of a NO-releasing NSAID derivative might carry with it a substantial dose of the NO-donor moiety, it was noted that nitroglycerin is rapidly inactivated in the liver, suggesting that NO-releasing NSAID derivatives should be relatively safe for use in humans and animals. See Smith, R. P., "Non-Steroidal Anti-inflammatory Drugs (NSAID) that Release Nitric Oxide (NO)", *Inflammopharmacology,* 3, 1995, 195-196.

Further investigations in the art established that NO-releasing NSAID derivatives act at the molecular level in low nanomolar concentrations to inhibit NO synthase induction without directly affecting enzyme activity. The nitroxybutylester derivatives of flurbiprofen, aspirin, ketoprofen, naproxen, diclofenac, and ketorolac were evaluated. See Cirino, G.; Wheeler-Jones, C. P. D.; Wallace, J. L.; Del Soldato, P.; and Baydoun, A. R.; "Inhibition of Inducible Nitric Oxide Synthase Expression by Novel Nonsteroidal Anti-inflammatory Derivatives with Gastrointestinal-Sparing Properties"; *British Journal of Pharmacology,* 117, 1996, 1421-1426. These same NO-releasing NSAID derivatives were also investigated and found to show markedly less ulcerogenic activity but comparable anti-inflammatory properties to their parent compounds in acute and chronic models of inflammation in the rat. See Del Soldato, P.; Cuzzolin, L.; Adami, A.; Conforti, A.; Crivellente, F.; and Benoni, G.; "Nitric Oxide-Releasing NSAIDs, a Novel Class of Safe and Effective Anti-inflammatory Agents", *Inflammopharmacology,* 4, 1996, 181-188.\

Comparative safety evaluations were made between flurbiprofen and nitroxybutylflurbiprofen in order to determine their effect and potency to uncouple mitochondrial oxidative phosphorylation, an early pathogenic event in NSAID enteropathy; to increase intestinal permeability, a transitional stage; and to cause macroscopic small intestinal damage. The results of the evaluations showed that NO-flurbiprofen was associated with significantly less macroscopic damage in the small intestine than flurbiprofen, but that NO-flurbiprofen was associated with mitochondrial damage *in vivo,* and that it caused similar increases in permeability of the small intestine as flurbiprofen. It was concluded that the beneficial effect of NO-flurbiprofen was exhibited in the later pathogenic stages of the damage. However, it was also noted that the significant decrease in the development of ulcers with NO-flurbiprofen was consistent with the suggestion that alterations in the microcirculation were the driving force in converting the biochemical damage of NSAIDs into ulcers. See Somasundaram, S.; Rafi, S.; Jacob, M.; Sigthorsson, G.; Mahmud, T.; Sherwood, R.; Price, A. B.; Macpherson, A.; Scott, D.; Wrigglesworth, J. M.; and Bjarnason, I.; "Intestinal Tolerability of Nitroxybutyl-Flurbiprofen in Rats", *Gut,* 40, 1997, 608-613.

In addition to reduced mucosal blood flow, the most important event in the pathogenesis of NSAID-induced gastric damage is the adherence of neutrophils to the vascular endothelium. Both of these actions are blocked by nitrogen oxide and NO-releasing NSAID derivatives. See Wallace, J. L. and Chin, B. C., "New Generation NSAIDs. the Benefits Without the Risks?", *Drugs of Today,* 33(6), 1997, 371-378.

None of the above-discussed articles from the technical literature describing the state of the art in any way suggests the unique nitric oxide releasing oxindole prodrugs of the present invention, nor do any of them create any reasonable expectation that the novel nitric oxide releasing oxindole prodrugs of the present invention will have the same properties as the NO-releasing NSAIDs described in said articles.

Matji, J. A, Alcaide, A.; and Corlay, S. L.; WO 94/04484 refers to nitric esters including nitroxybutyl esters of diclofenac, said to be useful for the treatment of anti-inflammatory conditions but without the adverse reactions typical of anti-inflammatory drugs. However, there is no suggestion of the unique nitric oxide releasing oxindole prodrugs of the present invention.

Arena, B., WO 94/12463 refers to nitric esters including nitroxybutyl esters of well known NSAIDs including carprofen, etodolac, flurbiprofen, indoprofen, indobufen, ketoprofen and suprofen, said to be useful for the treatment of anti-inflammatory conditions but without the adverse reactions typical of anti-inflammatory drugs. However, there is no suggestion of the unique nitric oxide releasing oxindole prodrugs of the present invention.

Del Soldato, P. and Sannicolo, F., WO 95/30641 refers to nitric esters including nitroxybutyl esters of well known NSAIDs including mesalamine, olsalazine, sulfasalazine, flunixin, ketoprofen, carprofen, tiaprofenic acid, suprofen, indoprofen, etodolac, fenoprofen, fenbufen, flurbiprofen, tolmetin, pranoprofen, bermoprofen, pemedolac, pirazolac, zaltoprofen, mofezolac, naproxen, loxoprofen, ibuprofen, ketorolac, tenidap, tenoxicam, piroxicam, nabumetone, indomethacin, meloxicam, ampiroxicam, bromfenac, and lornoxicam, said to be useful for the treatment of anti-inflammatory conditions but without the adverse reactions typical of anti-inflammatory drugs. None of the esters disclosed in WO 95/30641 would suggest the unique nitric oxide releasing oxindole prodrugs of the present invention. This disclosure includes that of esters of tenidap, which is referred to on page 26 thereof, along with a reference to derivatives thereof disclosed in Kadin US 4,556,672. The core nucleus of such anti-inflammatory compounds may be found in many of the nitric oxide releasing oxindole prodrugs of the present invention; however, the esters referred to in WO 95/30641 are not the same as those of the present invention. To the contrary, based on the discussion on pages 23, 26, 28 and 29 of WO 95/30641, said esters are of the type represented by the following formula: 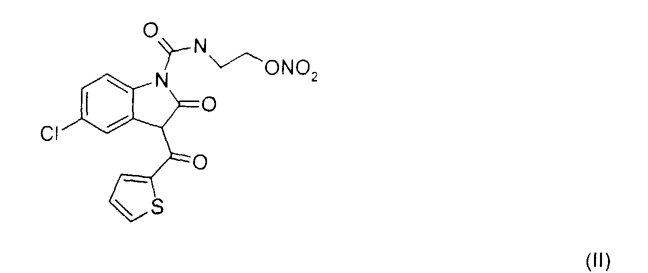 where the nitric oxide ester is formed with the 1-carboxamide substituent on the 2-oxo-1H-indole nucleus of the tenidap molecule. In a surprising departure from such a conventional approach to the arrangement of nitric oxide releasing esters, the nitric oxide releasing oxindole prodrugs of the present invention comprise an elaboration of the 3-enol moiety of the core nucleus. This unexpected structural difference may be seen by contrasting the compound of Formula (II) above with the following compound of Formula (III) 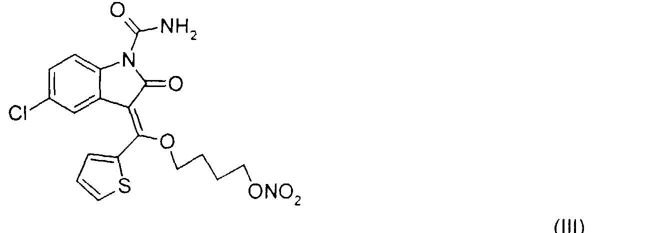 There is no teaching in WO 95/30641 that the nitric oxide releasing moiety should be attached as a prodrug fragment, or that the point of attachment of said moiety to the core nucleus of such compounds as tenidap should be at the 3-enol group.

Garvey, D. S.; Letts, L. G.; Renfroe, H. B.; and Tam, S. W.; WO 96/32946 refers to NSAIDs either linked to a nitric oxide group or co-administered with a nitric oxide donating group, which possess analgesic/anti-inflammatory properties but with a reduced potential for producing gastrointestinal lesions. Nitric oxide donors are said to include S-nitrosothiols, nitrites and N-oxo-N-nitrosamines, but nitric esters of the NSAIDs are specifically excluded (page 10). Tenidap is referred to specifically (pages 9, 75 and 83), but in the context of being co-administered with a separate nitric oxide donating compound. Accordingly, there is no suggestion of the unique nitric oxide releasing oxindole prodrugs of the present invention.

### SUMMARY OF THE INVENTION

The present invention relates to novel compositions of matter comprising a compound of Formula (I): 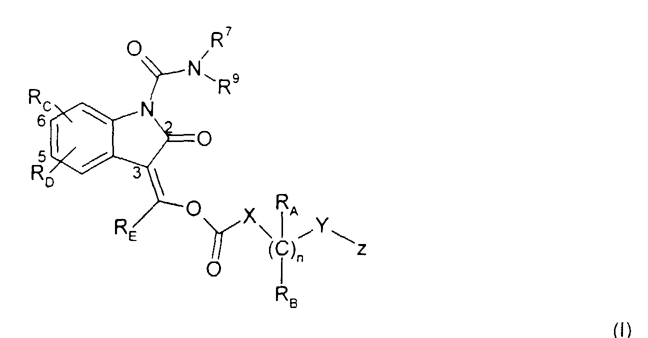 and to pharmaceutically acceptable salts thereof; wherein X is a covalent bond, -O-, -S-, or -N(R¹)- where R¹ is independently H or (C₁-C₄) alkyl wherein said alkyl may be straight or branched chain; R_{A} and R_{B} are members independently selected from the group consisting essentially of H; (C₁-C₄) alkyl; (C₃-C₆) cycloalkyl; phenyl; or taken together with the carbon atom to which they are attached, form a bridging (C₃-C₆) cycloalkyl moiety; wherein said alkyl may be straight or branched chain, and wherein said alkyl. cycloalkyl, phenyl and bridging cycloalkyl moieties are independently substituted with 0 to 2 R³, where R³ is a member selected from the group consisting essentially of F, Cl, Br, CF₃, and (C₁-C₄) alkoxy; n is an integer selected from 1 through 6, inclusive; Y is a covalent bond, -O-, -S-, or -N-; Z is -NO or -NO₂; R_{C} is a member independently selected from the group consisting essentially of H, F, Cl, Br, (C₁-C₄) alkyl, (C₃-C₇) cycloalkyl, (C₁-C₄) alkoxy, (C₁-C₄) alkylthio, CF₃, (C₁-C₄) alkylsulfinyl, (C₁-C₄) alkylsulfonyl, NO₂, phenyl, (C₂-C₄) alkanoyl, benzoyl, thenoyl, (C₂ -C₄) alkanamido, benzamido, and N,N-di-(C₁-C₃) alkylsulfamoyl; R_{D} is a member independently selected from the group consisting essentially of H, F, Cl, Br, (C₁-C₄) alkyl, (C₃-C₇) cycloalkyl, (C₁-C₄) alkoxy, (C₁-C₄) alkylthio, and CF₃; or R_{C} and R_{D} when taken together are a 4,5-, 5,6- or 6,7-methylenedioxy group or a 4,5-, 5,6- or 6,7-ethylenedioxy group; or R_{C} and R_{D} when taken together and when attached to adjacent carbon atoms, form a divalent radical D, wherein D is selected from the group consisting essentially of: 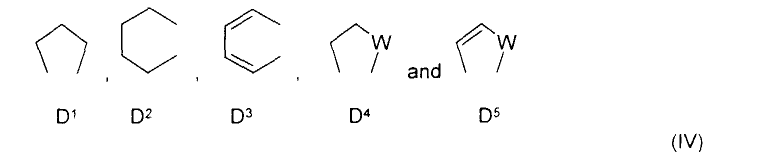 wherein W is O or S; R_{E} is a member independently selected from the group consisting essentially of (C₁-C₆) alkyl, (C₃-C₇) cycloalkyl, (C₄-C₇) cycloalkenyl, phenyl, phenyl(C₁-C₃) alkyl, phenoxy(C₁-C₃) alkyl, thiophenoxy(C₁-C₃) alkyl, naphthyl, bicyclo[2.2.1]heptan-2-yl, bicyclo[2.2 1]hept-5-en-2-yl, and -(CH₂)ₘ-Q-R²; wherein said phenyl, phenylalkyl and phenoxyalkyl moieties are independently substituted with 0 to 2 R⁵, where R⁵ is a member selected from the group consisting essentially of F, Cl, Br, CF₃, (C₁-C₄) alkyl and (C₁-C₄) alkoxy; m is 0, 1 or 2; Q is a divalent radical derived from a compound independently selected from the group consisting essentially of furan, thiophene, pyrrole, pyrazole, imidazole, thiazole, isothiazole, oxazole, isoxazole, 1,2,3-thiadiazole, 1,3,4-thiadiazole, 1,2,5-thiadiazole, tetrahydrofuran, tetrahydrothiophene, tetrahydropyran, tetrahydrothiopyran, pyridine, pyrimidine, pyrazine, benzo[b]furan and benzo[b]thiophene; and R² is a member selected from the group consisting essentially of H, F, Cl, Br, CF₃, (C₁-C₃)alkyl, and (C₁-C₃)alkoxy; and R⁷ and R⁹ are independently selected from the group consisting essentially of hydrogen, (C₁-C₃)alkyl, -C(=O) (C₁-C₃) alkyl, phenyl, and -C(=O)N(R⁸)(R¹⁰), where R⁸ and R¹⁰ are independently selected from the group consisting essentially of hydrogen, (C₁-C₃) alkyl, -C(=O) (C₁-C₃) alkyl, and phenyl .

The present invention further relates to a compound of Formula (I) wherein X is a covalent bond, -O-, or -N(R¹)-; R_{A} and R_{B} are independently H, methyl, ethyl, phenyl, or a bridging cyclopentyl moiety when taken together with the carbon atom to which they are attached; n is an integer selected from 3 through 6, inclusive; Y is -O- or -S-; and Z is -NO or -NO₂.

The present invention still further relates to a compound of Formula (I) wherein X is a covalent bond; R_{A} and R_{B} are independently H, methyl, or phenyl, where methyl and phenyl are optionally mono-substituted by F, Cl, or methoxy; n is an integer selected from 4 and 5; Y is -O-; and Z is -NO₂.

The present invention yet still further relates to a compound of Formula (I) wherein X is a covalent bond; R_{A} and R_{B} are both H; n is the integer 4; Y is -O-; and Z is -NO₂.

The present invention also relates to the above-mentioned preferred embodiments comprising compounds having the particular definitions of X, R_{A} and R_{B}, n, Y and Z taken together with the especially preferred embodiments comprising compounds having the particular definitions of R_{C}, R_{D}, and R_{E} recited in the following paragraph. In these various preferred embodiments it is preferred that R⁷ and R⁹ are both hydrogen or methyl, or that one of R⁷ and R⁹ is hydrogen and the other is methyl, phenyl, or -C(=O)N(R⁸)(R¹⁰), where R⁸ and R¹⁰ are preferably both hydrogen or methyl, or one of R⁸ and R¹⁰ is hydrogen and the other is methyl.

The present invention preferably relates to compounds of Formula (I) having the above-recited particular definitions of X, R_{A} and R_{B}, n, Y and Z, wherein also R_{D} is preferably hydrogen, R_{C} is preferably a member selected from the group consisting essentially of 5-Cl, 6-Cl, 5-F, 6-F, 5-CF₃ and 6-CF₃; and R_{E} is a member selected from the group consisting essentially of benzyl for which the optional substituent R⁵ is F, Cl, or CF₃, and of 2-furyl, 2-thienyl, (2-furyl)methyl, and (2-thienyl)methyl for each of which R² is H, F, Cl, CF₃, CH₃ or OCH₃. In another preferred embodiment R_{C} is a member selected from the group consisting of 5-Cl and 5-F, R_{D} is a member selected from the group consisting essentially of 6-Cl and 6-F, and R_{E} is a member selected from the group consisting essentially of benzyl, 2-furyl, 2-thienyl, 5-chloro-2-thienyl and 5-trifluoromethyl-2-thienyl.

In addition to the above-described compounds of Formula (I), the present invention relates to a method of treating or preventing pain, inflammation, fever, or gastrointestinal lesions in a patient in need of such treatment, comprising administering to said mammal a therapeutically effective amount of a compound of Formula (I). The present invention further relates to a non-enteropathogenic method of treating osteoarthritis, an inflammatory disease or condition, or a degenerative joint disease or condition, in a patient in need of such treatment, comprising administering to said patient a therapeutically effective but non-enteropathogenic amount of a compound of Formula (I). The present invention still further relates to a method of modifying an osteoarthritic, inflammatory, or degenerative joint disease or condition in a patient in need of such treatment, comprising administering to said patient an amount of a compound of Formula (I) which is therapeutically effective in halting or reversing the early stages of degeneration and decline in said patient's body tissues, organs, and basic functions associated with said disease or condition, whereby the outcome of said disease or condition is favorably affected.

The present invention is viewed as relating to, and is also intended to include within its scope, a non-enteropathogenic method of treating and modifying an osteoarthritic, inflammatory, or degenerative joint disease or condition in a patient in need of such treatment, comprising administering to said patient a non-enteropathogenic amount of a compound of Formula (I) which is therapeutically effective in halting or reversing the early stages of degeneration and decline in said patient's body tissues, organs, and basic functions associated with said disease or condition, whereby the outcome of said disease or condition in said patient is favorably affected. Said patient will most typically be a mammal, preferably a livestock animal, a companion animal, or a human.

The present invention also relates to analgesic, anti-inflammatory, and anti-pyretic pharmaceutical compositions comprising a pharmaceutically acceptable carrier and a compound of Formula (I). Further, the present invention relates to non-enteropathogenic pharmaceutical compositions for use in modifying the course of an osteoarthritic, inflammatory, or degenerative joint disease or condition in a patient in need of such treatment, comprising a non-enteropathogenic amount of a compound of Formula (I) which is therapeutically effective in halting or reversing the early stages of degeneration and decline in said patient's body tissues, organs, and basic functions associated with said disease or condition, together with a pharmaceutically acceptable carrier for said compound of Formula (I).

### DETAILED DESCRIPTION OF THE INVENTION

A central objective of the present invention is provide a highly effective treatment for pain, inflammation and fever occurring in mammals in need of such treatment, by the administration of anti-inflammatory therapeutic agents to such mammals which do not concomitantly possess toxic properties with respect to the gastrointestinal tract of said mammals. It is a further objective of the present invention to provide a method of modifying an osteoarthritic, inflammatory, or degenerative joint disease or condition by favorably affecting the outcome thereof. Satisfying this objective involves preventing or reducing those disease processes which cause tissue damage as a concomitant of inflammation. Amelioration of symptoms alone is not the goal with regard to this embodiment of the present invention. Rather, the purpose is to achieve disruption of the underlying mechanisms causing irreparable and irreversible tissue damage.

The novel compounds of the present invention not only possess excellent anti-inflammatory activity as demonstrated in accepted animal models of acute and chronic inflammation, but at the same time produce markedly less gastric injury in the patient being treated, when administered on both an acute and a chronic basis. Indeed, the NO-releasing compounds of the present invention inhibit the release of reactive oxygen metabolites and proteases from white blood cells within the gastrointestinal microcirculation, and prevent a reduction in such circulation, thereby producing a significant acceleration in the healing of any gastric ulcers which may form.

The gastric toxicity of most non-steroidal anti-inflammatory drugs has been known in the art for some time, and the various modes of action by which it is hypothesized that this gastric toxicity occurs have been the subject of innumerable studies reported in the technical literature. However, the pharmacological action of these NSAIDs is very complex and still not completely understood.

Approaches to preventing, hindering or reversing such gastric toxicity have included the exploration of compounds which are nitric oxide donors, *i.e*., compounds which directly donate, release or transfer nitrogen monoxide, i.e., nitric oxide (NO), preferably as a charged species, *e.g*., nitrosonium species, to other molecules and which act to prevent, reduce, or reverse the gastrointestinal, renal and other toxicities induced by most NSAIDs. Such NO-releasing moieties may be directly or indirectly linked to such NSAIDs, as NO-donating moieties forming part of the overall NSAID molecular structure, or as separate NO-donating compounds which are co-administered with said NSAIDs. The therapeutic usefulness of such NO-donating moieties, whether separate entities or part of the structure of an existing NSAID, is based on the rationale that since NO maintains gastric mucosal blood flow and prevents leukocyte adherence within the gastric microcirculation, that NO-donating moieties might be reasonably expected to counteract the detrimental effects of cyclooxygenase suppression.

The novel compounds of the present invention are believed to owe their particular therapeutic usefulness in maintaining mucosal blood flow and preventing leukocyte adherence within the gastric microcirculation to their unique chemical structure in which the nitric oxide ester is formed with the hydroxy-2-thienylmethylene moiety rather than the 1-carboxamide substituent on the basic 2-oxo-1H-indole nucleus. Two important compounds of this type are tenidap and ilonidap. Tenidap, a known NSAID compound, may be represented by the following Formula (V): 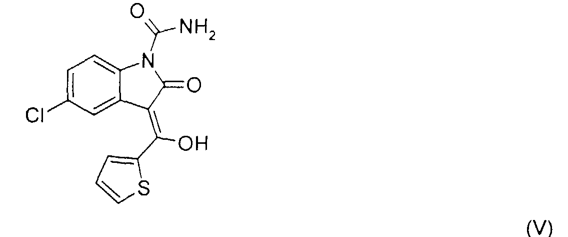 Ilonidap, also a known NSAID compound, may be represented by the following Formula (VI):

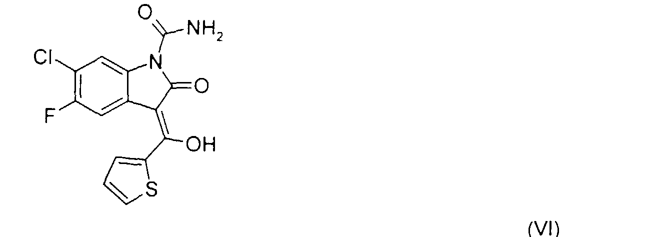

Accordingly, the present invention relates to a compound of Formula (I): 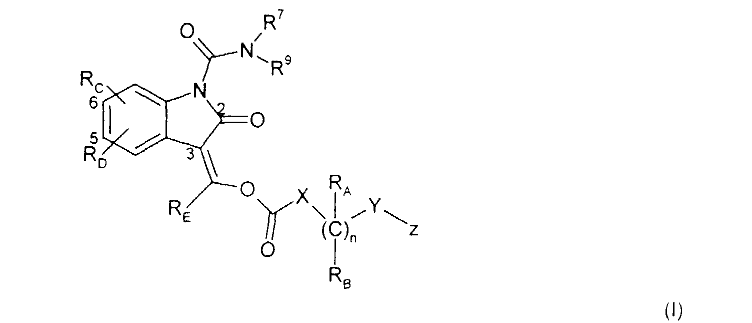 and to pharmaceutically acceptable salts thereof; wherein
X is a covalent bond, -O-, -S-, or -N(R¹)- where R¹ is independently H or (C₁-C₄) alkyl wherein said alkyl may be straight or branched chain;
R_{A} and R_{B} are members independently selected from the group consisting essentially of H; (C₁-C₄) alkyl; (C₃-C₆) cycloalkyl; phenyl; or taken together with the carbon atom to which they are attached, form a bridging (C₃-C₆) cycloalkyl moiety; wherein said alkyl may be straight or branched chain, and wherein said alkyl, cycloalkyl, phenyl and bridging cycloalkyl moieties are independently substituted with 0 to 2 R³, where R³ is a member selected from the group consisting essentially of F, Cl, Br, CF₃, and (C₁-C₄) alkoxy;
n is an integer selected from 1 through 6, inclusive;
Y is a covalent bond, -O-, -S-, or -N-;
Z is -NO or -NO₂;
R_{C} is a member independently selected from the group consisting essentially of H, F, Cl, Br, (C₁-C₄) alkyl, (C₃-C₇) cycloalkyl, (C₁-C₄) alkoxy, (C₁-C₄) alkylthio, CF₃, (C₁-C₄) alkylsulfinyl, (C₁-C₄) alkylsulfonyl, NO₂, phenyl, (C₂ -C₄) alkanoyl, benzoyl, thenoyl, (C₂ -C₄) alkanamido, benzamido, and N,N-di-(C₁-C₃) alkylsulfamoyl;
R_{D} is a member independently selected from the group consisting essentially of H, F, Cl, Br, (C₁-C₄) alkyl, (C₃-C₇) cycloalkyl, (C₁-C₄) alkoxy, (C₁-C₄) alkylthio, and CF₃; or
R_{C} and R_{D} when taken together are a 4,5-, 5,6- or 6,7-methylenedioxy group or a 4,5-, 5,6- or 6,7-ethylenedioxy group; or
R_{C} and R_{D} when taken together and when attached to adjacent carbon atoms, form a divalent radical D, wherein D is selected from the group consisting essentially of

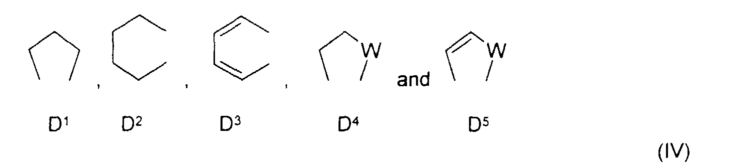

wherein W is O or S;
R_{E} is a member independently selected from the group consisting essentially of (C₁-C₆) alkyl, (C₃ -C₇) cycloalkyl, (C₄ -C₇) cycloalkenyl, phenyl, phenyl(C₁-C₃) alkyl, phenoxy(C₁-C₃) alkyl, thiophenoxy(C₁-C₃) alkyl, naphthyl, bicyclo[2 2.1]heptan-2-yl, bicyclo[2.2.1]hept-5-en-2-yl, and -(CH₂)ₘ-Q-R²; wherein said phenyl, phenylalkyl and phenoxyalkyl moieties are independently substituted with 0 to 2 R⁵, where R⁵ is a member selected from the group consisting essentially of F, Cl, Br, CF₃, (C₁-C₄)alkyl and (C₁-C₄) alkoxy; m is 0, 1 or 2; Q is a divalent radical derived from a compound independently selected from the group consisting essentially of furan, thiophene, pyrrole, pyrazole, imidazole, thiazole, isothiazole, oxazole, isoxazole, 1,2,3-thiadiazole, 1,3,4-thiadiazole, 1,2,5-thiadiazole, tetrahydrofuran, tetrahydrothiophene, tetrahydropyran, tetrahydrothiopyran, pyridine, pyrimidine, pyrazine, benzo[b]furan and benzo[b]thiophene; and R² is a member selected from the group consisting essentially of H, F, Cl, Br, CF₃, (C₁-C₃)alkyl, and (C₁-C₃) alkoxy; and
R⁷ and R⁹ are independently selected from the group consisting essentially of hydrogen, (C₁-C₃) alkyl, -C(=O) (C₁-C₃)alkyl, phenyl, and -C(=O)N(R⁸)(R¹⁰), where R⁸ and R¹⁰ are independently selected from the group consisting essentially of hydrogen, (C₁-C₃) alkyl, -C(=O) (C₁-C₃) alkyl, and phenyl.

The term "alkyl" as used with reference to the moieties "R_{A}" and "R_{B}", as well as in other contexts throughout the instant specification, and whether used alone or in combination, refers to a straight-chain or branched chain alkyl radical containing the indicated number of carbon atoms, from 1 to 4. Examples of such radicals include, but are not limited to, methyl, ethyl, *n*-propyl, *iso*-propyl, *n*-butyl, *iso*-butyl, *sec*-butyl, and *tert*-butyl.

The term "alkoxy" as used with reference to the substituents which may be attached to any of the groups which define "R_{A}" and "R_{B}", as well as in other contexts throughout the instant specification, and whether used alone or in combination, refers to an alkyl ether radical, wherein the term "alkyl" is as defined above. Examples of suitable alkyl ether radicals include, but are not limited to, methoxy, ethoxy, *n*-propoxy, *iso*-propoxy, *n*-butoxy, *iso*-butoxy, *sec*-butoxy, and *tert*-butoxy.

The term "cycloalkyl" as used with reference to the moieties "R_{A}" and "R_{B}", as well as in other contexts throughout the instant specification, and whether used alone or in combination, refers to a cyclic alkyl radical containing from 3 to 6 carbon atoms. Examples of such cycloalkyl radicals include, but are not limited to, cyclopropyl, cyclobutyl, cyclopentyl, and cyclohexyl.

Preferred embodiments of the present invention comprise compounds of Formula (I) wherein X is a covalent bond, -O-, or -N(R¹)-; R_{A} and R_{B} are independently selected from the group consisting essentially of hydrogen; methyl; ethyl; propyl; *tert*-butyl; cyclopropyl; cyclohexyl; phenyl; phenyl substituted by 1 or 2 R³ where R³ is F, Cl, CF₃-, or CH₃O-; and a bridging cyclopentyl moiety when taken together with the carbon atom to which they are attached; n is an integer selected from 3 through 6, inclusive; Y is -O- or -S-; and Z is -NO or -NO₂. More preferred embodiments of the present invention comprise a compound of Formula (I) wherein X is a covalent bond; R_{A} and R_{B} are independently selected from the group consisting essentially of hydrogen; propyl; *tert*-butyl; cyclopropyl; cyclohexyl; phenyl; phenyl substituted by 1 or 2 of F or CH₃O-; n is an integer selected from 4 and 5; Y is -O-; and Z is -NO₂. Still more preferred embodiments of the present invention comprise a compound of Formula (I) wherein X is a covalent bond; R_{A} and R_{B} are both hydrogen; n is the integer 4; Y is -O-; and Z is -NO₂.

Still further preferred embodiments of the present invention comprise the above-mentioned preferred embodiments comprising compounds having the particular definitions of X, R_{A} and R_{B}, n, Y and Z therein set out, taken together with the especially preferred embodiments comprising compounds having the particular definitions of R_{C}, R_{D}, and R_{E} recited further below. In these various especially preferred embodiments it is preferred that R⁷ and R⁹ are both hydrogen; or they are both methyl; or one of R⁷ and R⁹ is hydrogen and the other is independently selected from the group consisting essentially of methyl; phenyl; and -C(=O)N(R⁸)(R¹⁰), where R⁸ and R¹⁰ are both hydrogen, or they are both methyl, or one of R⁸ and R¹⁰ is hydrogen and the other is methyl.

Thus, especially preferred embodiments of the present invention comprise compounds of Formula (I) having the above-recited particular definitions of X, R_{A} and R_{B}, n, Y and Z, wherein also R_{D} is preferably hydrogen, R_{C} is preferably a member selected from the group consisting essentially of 5-Cl, 6-Cl, 5-F, 6-F, 5-CF₃ and 6-CF₃; and R_{E} is a member selected from the group consisting essentially of benzyl for which the optional substituent R⁵ is F, Cl, or CF₃, and of 2-furyl, 2-thienyl, (2-furyl)methyl, and (2-thienyl)methyl for each of which R² is H, F, Cl, -CF₃, -CH₃ or -OCH₃. In another especially preferred embodiment R_{C} is a member selected from the group consisting essentially of 5-Cl and 5-F, R_{D} is a member selected from the group consisting essentially of 6-Cl and 6-F, and R_{E} is a member selected from the group consisting essentially of benzyl, 2-furyl, 2-thienyl, 5-chloro-2-thienyl and 5-trifluoromethyl-2-thienyl.

The moiety R_{A}-(C)ₙ-R_{B} may be, and is preferably, repeated in accordance with the integer which is selected as the meaning for "n". It will be understood that the groups R_{A} and R_{B} appear as substituents on each carbon atom of the alkyl chain defined by "(C)ₙ". Accordingly, the moiety R_{A}-(C)ₙ-R_{B} may have two or more substituents defined by R_{A} and R_{B} and these may occur at any point of the alkyl chain defined by "(C)ₙ". For most or all of said carbon atoms, however, it is preferred that both R_{A} and R_{B} be H.

In those instances where X is -O- or -S-; n is an integer selected from 3 to 5; and R_{A} and R_{B} are independently H; (C₁-C₄) alkyl; (C₃-C₆) cycloalkyl; bridging (C₃-C₆) cycloalkyl;or phenyl; compounds of Formula (I) having the bridging moieties represented by the following partial Formulas (Ia) - (If) are within the scope of the present invention:

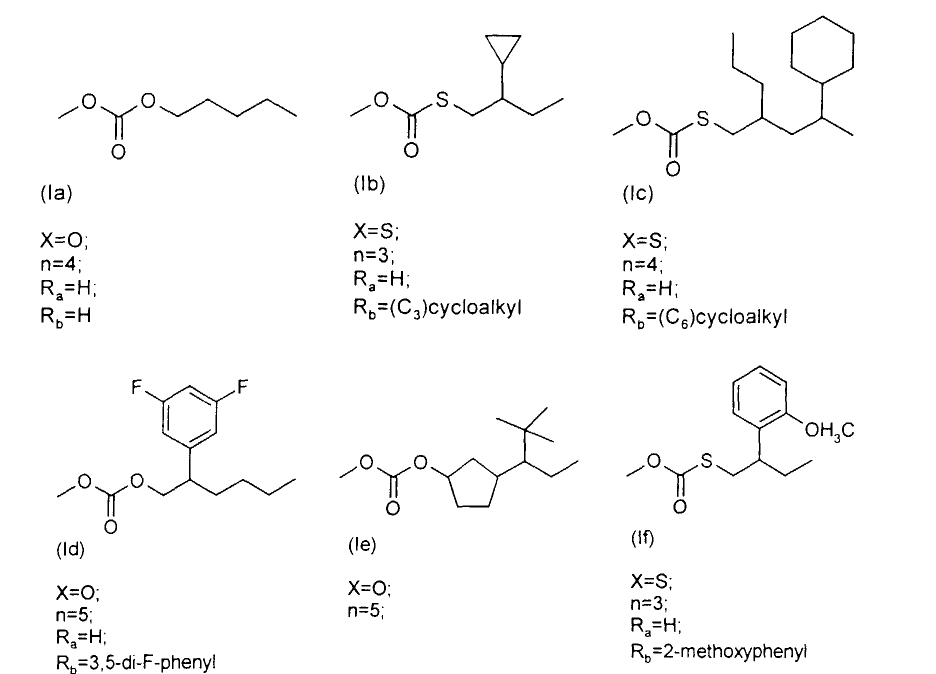

In the compounds of Formula (I) it is preferred that X is a covalent bond, -O-, or NR¹ where R¹ is independently H, methyl or ethyl, and preferably R¹ is H. More preferably, X is a covalent bond. It is also preferred that n is an integer selected from 3 through 5 inclusive, and more preferably n is the integer 4. Taking these preferred definitions into account, compounds of Formula (I) having the NO-donating moiety side chains represented by the following partial Formulas (Ig) - (Ik), where the "Y-Z" group is the preferred nitric oxide ester: "-O-NO₂", are within the scope of the present invention:

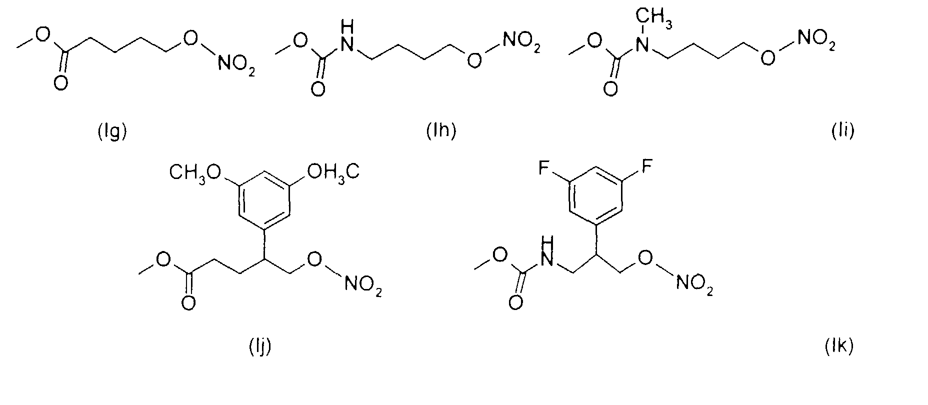

In addition to -O-, the Y group may also be -S-, resulting in S-nitrosylated enol ester forms of the NO-donating moiety side chain for the compounds of Formula (I), which may be represented by the following partial Formulas (II) and (Im):

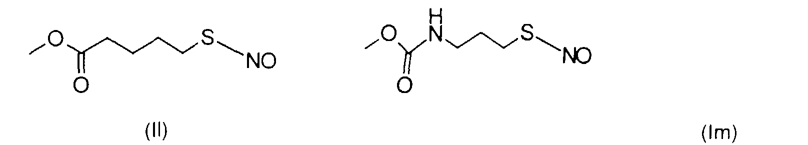

In accordance with the above-described preferred embodiments and especially preferred embodiments, there is included within the scope of the present invention the following specific compounds illustrated in the following Table (I):

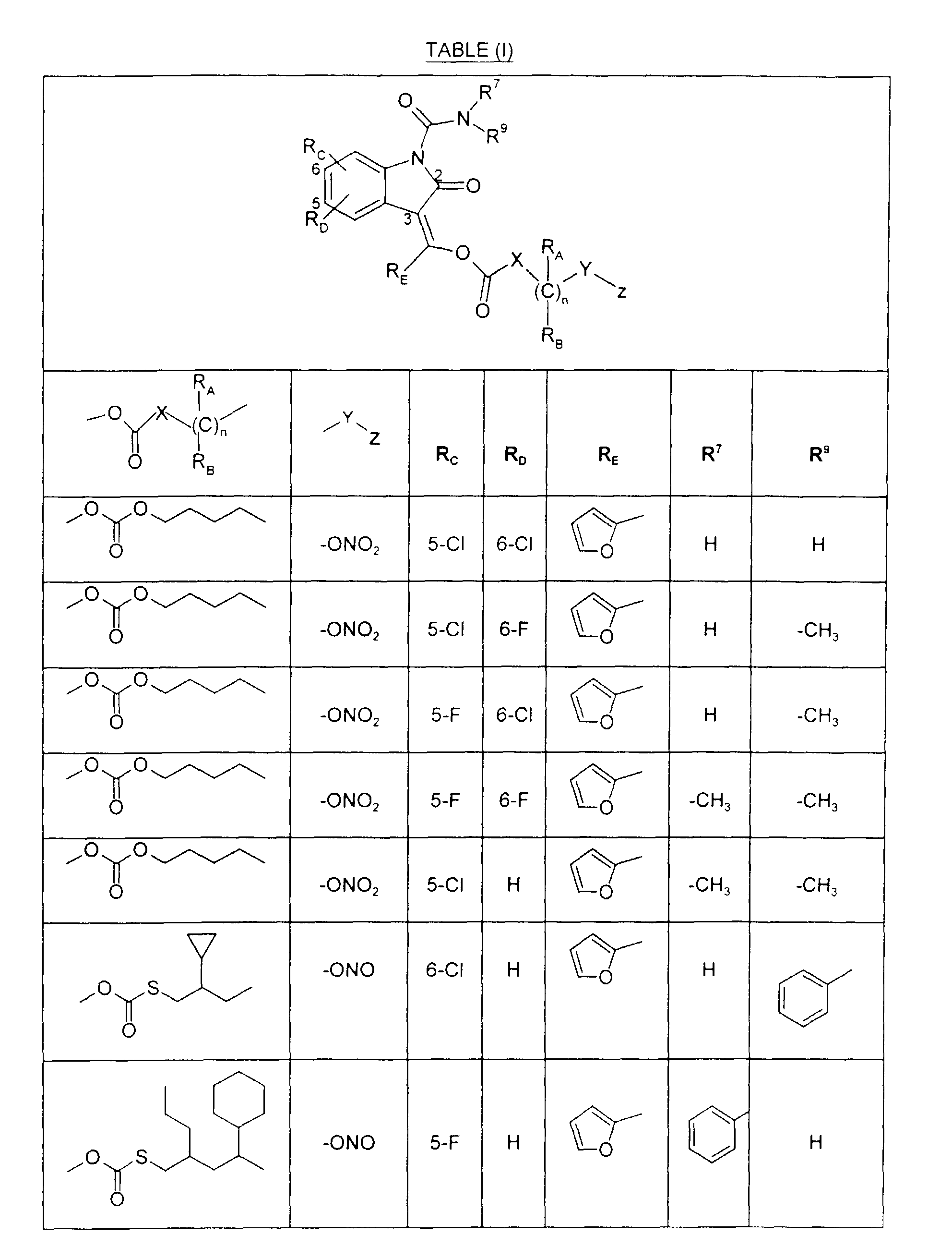

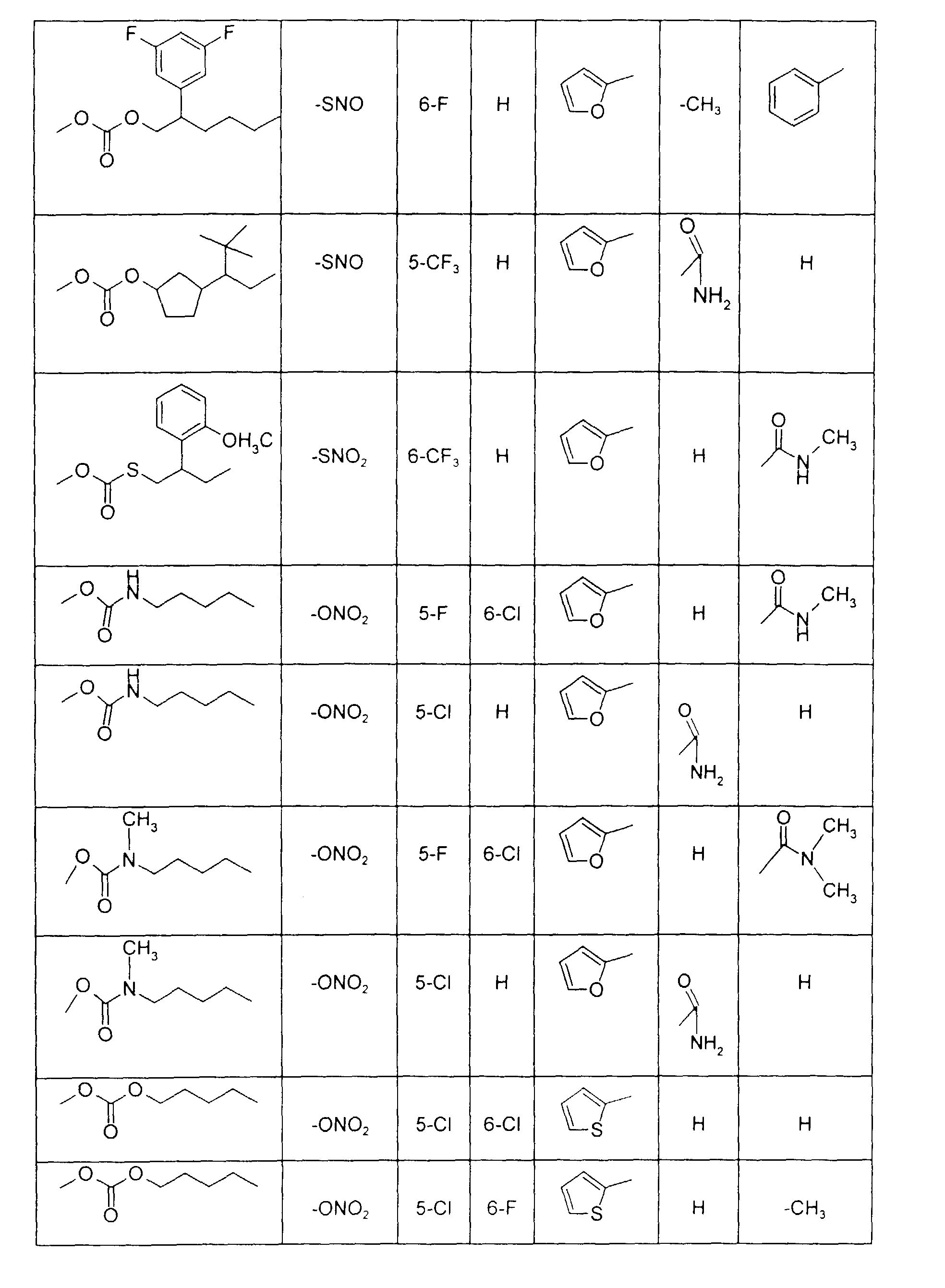

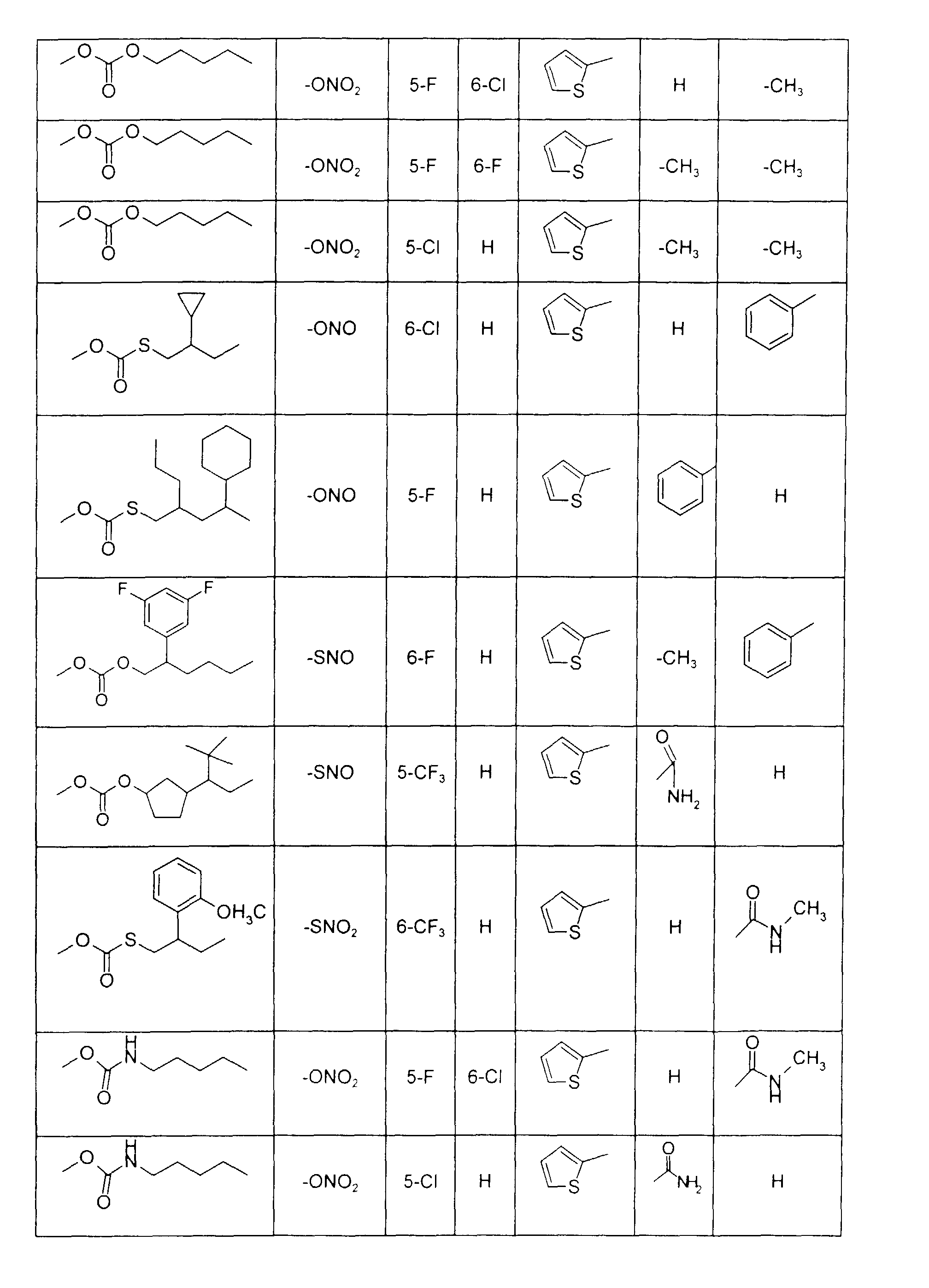

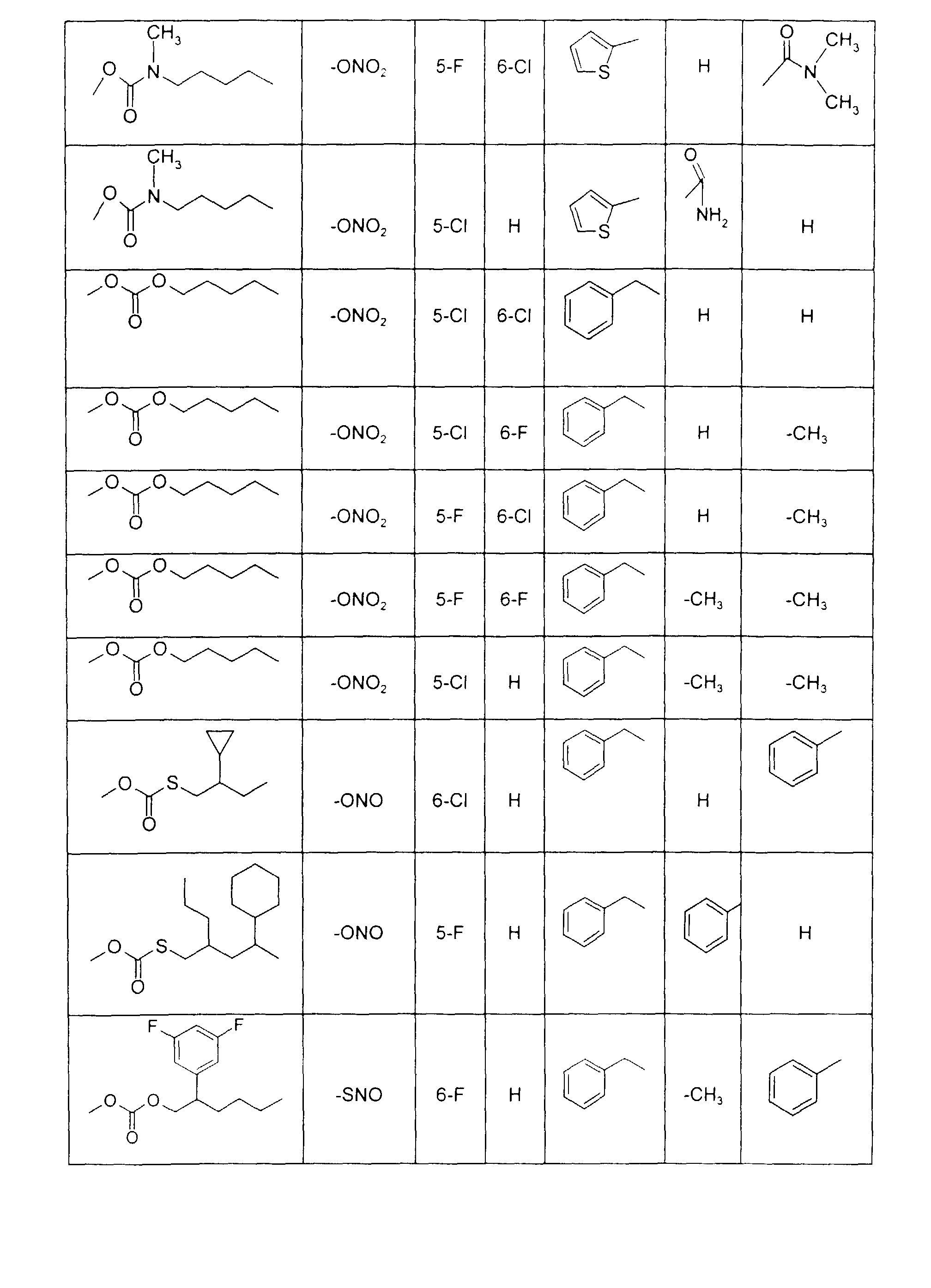

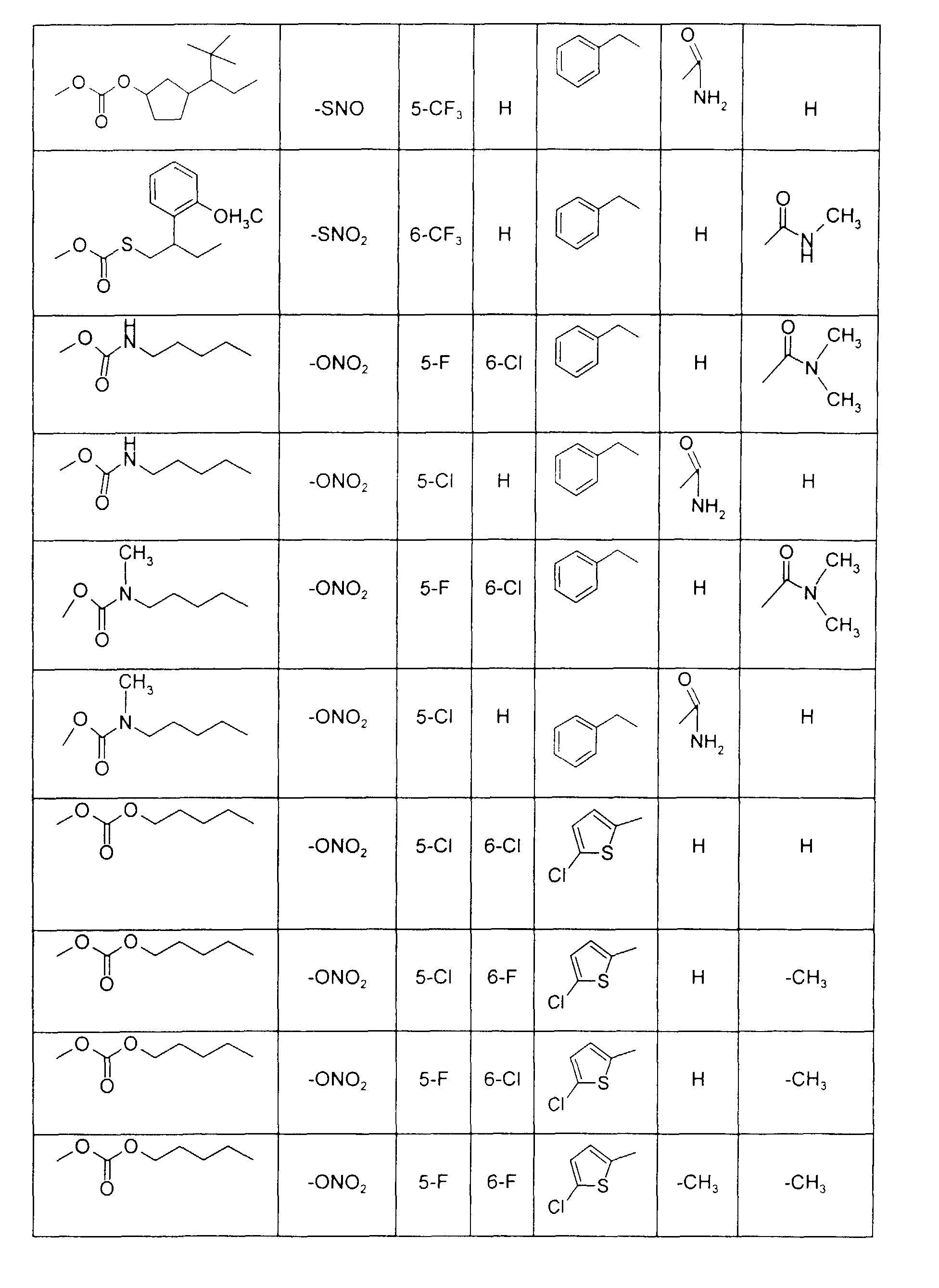

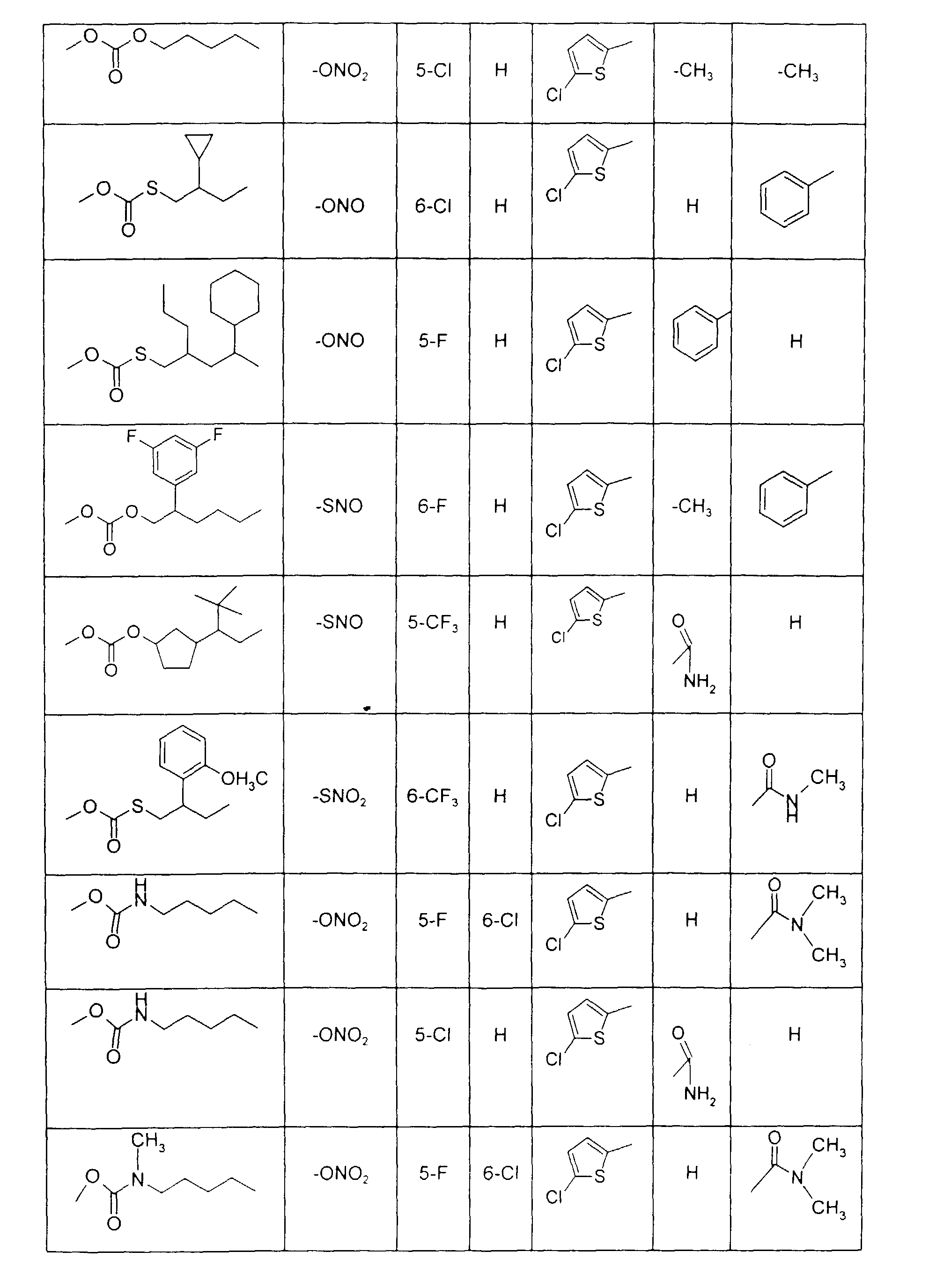

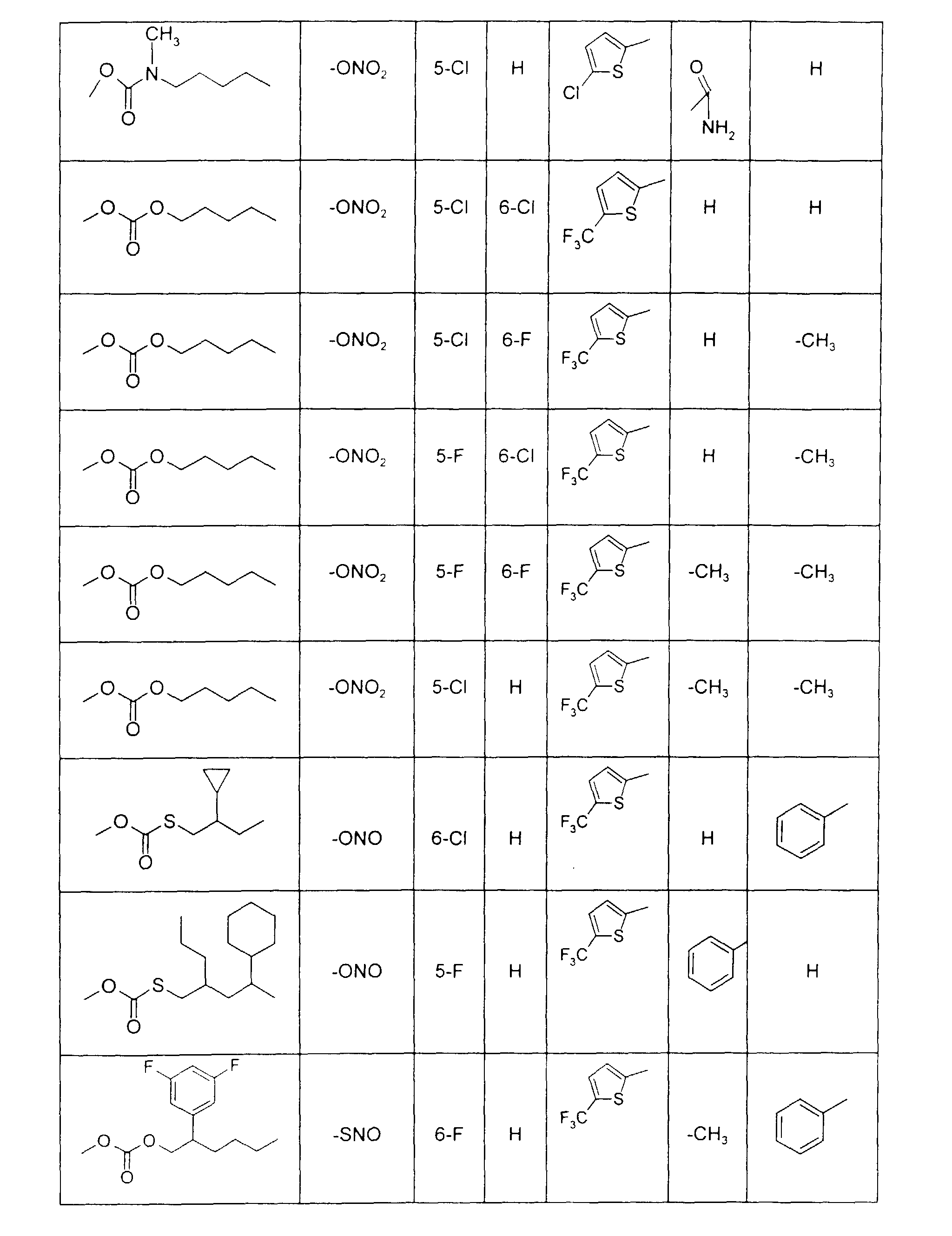

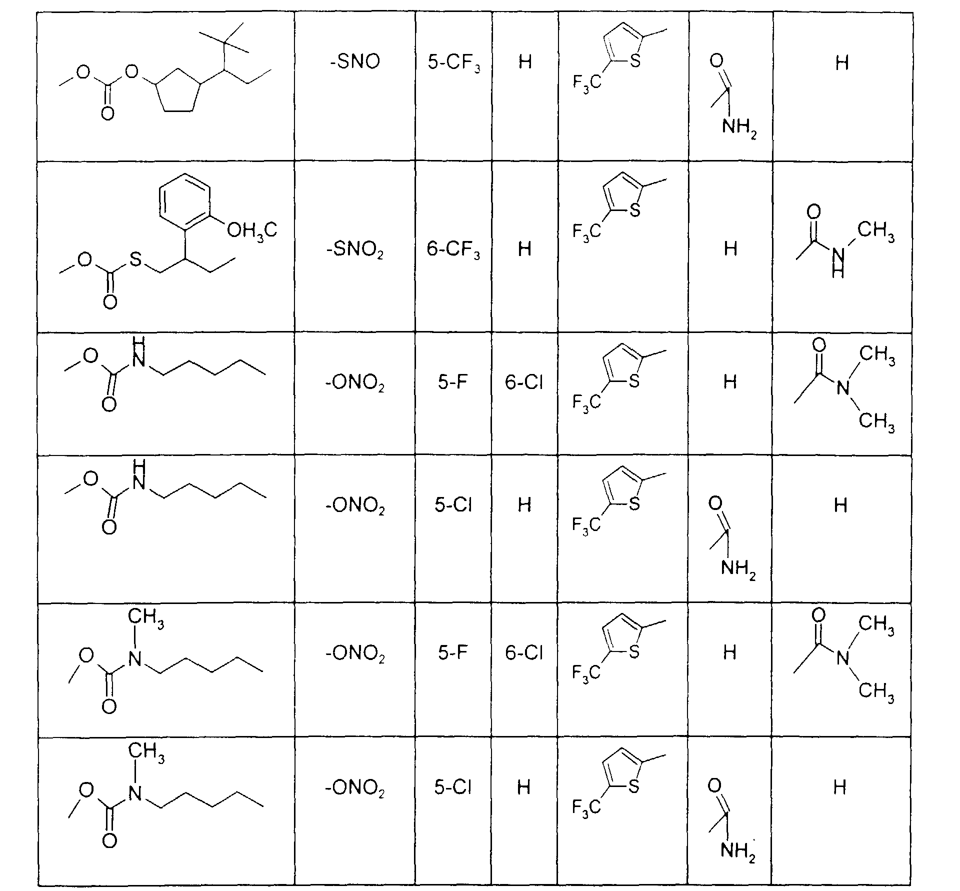

Included within the scope of the present invention are the pharmaceutically acceptable derivatives of the compounds of Formula (I). The expression "pharmaceutically acceptable derivative" as used in the instant specification denotes any pharmaceutically acceptable salt of a compound of Formula (I). Further included within the scope of the present invention is any other compound which, upon administration to a patient, is capable of directly or indirectly providing a compound of Formula (I). Such compounds are recognized as prodrugs, and a number of established procedures are available for preparing such prodrug forms of the compounds of Formula (I).

The expression "treating or preventing", as used herein with regard to the administration of the compounds of the present invention for therapeutic purposes to patients having osteoarthritic, inflammatory, or degenerative joint diseases and conditions is intended to denote both the therapeutic objective of said administration as well as the therapeutic results actually achieved by said administration. The extent of therapy accomplished by administration of the compounds of the present invention may range from palliative amelioration of symptoms to a significant diminishing of the course of the disease or condition involved, and beyond to active treatment of said disease or condition, including a reversal of the disease process itself which is present.

It is known that 3-substituted-2-oxindole-1-carboxamides, from which the nitric oxide releasing oxindole prodrugs of the present invention are for the most part derived, are useful as anti-inflammatory and analgesic agents, as described in Kadin US 4,556,672, already mentioned further above. In addition to these therapeutically useful biological activities, said 3-substituted-2-oxindole-1-carboxamides also inhibit the biosynthesis of IL-1, independent of their lipoxygenase inhibiting activity, thus making them useful in treating IL-1 mediated disorders and dysfunctions such as certain disorders of bone and connective tissue metabolism and dysfunctions of the immune system in mammals.

As described in more detail in Otterness US 4,861,794, which is incorporated herein by reference in its entirety, such bone metabolism disorders include, but are not limited to osteoarthritis; such connective tissue metabolism disorders include but are not limited to periodontal disease and tissue scarring; and IL-1 mediated immune dysfunctions include, but are not limited to, allergy and psoriasis. It is intended that the scope of the present invention include such disorders and dysfunctions as those disclosed in Otterness 4,861,794, as well as others that would be apparent to the artisan, within the reach of those diseases and conditions whose progress can be favorably modified using the nitric oxide releasing oxindole prodrugs of Formula (I). In the preferred embodiments of the present invention, nevertheless, the primary focus of such disease modification is with respect to those diseases and conditions which are precursors or sequelae of, or otherwise closely associated with, osteoarthritis, inflammatory diseases and conditions, and joint degeneration diseases and conditions, as described further herein.

The higher or highest degrees of therapeutic effectiveness result in the prevention of any injury, damage, deterioration, or loss of body tissues or organs and basic body functions subsequent to the early stages of degeneration and decline in said body tissues or organs and basic body functions at the onset of the osteoarthritic, inflammatory, or degenerative joint disease or condition involved

The expression "the early stages of degeneration and decline in body tissues or organs and basic body functions" is intended to mean the very beginning of the initial pathologic changes in said body tissues or organs and basic body functions which define and are the result of an osteoarthritic, inflammatory, or degenerative joint disease process or condition Particular tissues and organs involved in these types of diseases and conditions are joints and associated membranes and fibrous connective tissue including especially bone, subchondral bone, cartilage, ligaments and tendons The basic body functions involved in these types of diseases and conditions include especially sequestration and destruction of injurious agents and injured tissue; dilatation of arterioles, capillaries and venules with increased permeability, and blood flow; extravasation and exudation of fluids and plasma proteins; and leukocytic migration and infiltration into the inflammatory focus.

Pathologic changes in the above-mentioned tissues and organs from those present before the onset of said disease processes or conditions, include changes in the composition and cohesiveness; form and makeup; rigidity, strength, resilience, elasticity, conformational integrity and stability, density, tensile strength and other attributes of regular and established tissues and organs of this type, which result in degradation and a decline in the beneficial and necessary properties characterizing said tissues and organs. Further pathologic changes from those present before the onset of said disease processes and conditions, include abundance and location of said tissues and organs throughout the body; viability and regenerative capability on both a micro- and macro-level; and the ability to successfully resist various kinds of external stresses including mechanical force and invasion by microorganisms of said tissues and organs, which result in degradation and a decline in the beneficial and necessary properties characterizing said tissues and organs.

Pathologic changes with respect to body functions are those which inherently arise from the changes above-described with respect to said tissues and organs, and which also, consequently, result in a degradation and decline in the beneficial and necessary performance which characterizes the normal and proper operation of said body functions. These pathologic changes, both with regard to tissues or organs and with respect to body functions, especially include improper repair of the above-discussed early stages of degeneration and decline.

The term "patient" as used above and throughout the instant specification, refers to animals, particularly mammals, and especially livestock animals, companion animals, and humans. And where the term "cell" is used it refers to animal, particularly mammalian cells, including cells of humans, livestock animals, and companion animals, unless otherwise specified.. As used herein the terms "animal" and "animals" refer to all members of the animal kingdom and the primary divisions thereof which satisfy the other requirements imposed by the present invention with regard to nitric oxide releasing oxindole prodrugs having therapeutic utility with respect thereto. All pertinent phyla and subdivisions thereof are included, *e.g.,* the subphylum *Vertebrata* which includes classes of mammals *(Mammalia),* birds *(Aves),* reptiles *(Reptilia),* amphibians *(Amphibia)* and fishes *(Osteichthyes)*.

Particularly important animals and groups or types of animals which may benefit from treatment with the nitric oxide releasing oxindole prodrugs of the present invention are domesticated quadrupeds referred to herein as "livestock animals", which includes those being raised for meat and various byproducts, e.g., a bovine animal including cattle and other members of the genus *Bos,* a porcine animal including domestic swine and other members of the genus *Sus,* an ovine animal including sheep and other members of the genus *Ovis,* domestic goats and other members of the genus *Capra;* domesticated quadrupeds being raised for specialized tasks such as use as a beast of burden, *e.g.,* an equine animal including domestic horses and other members of the family Equidae, genus *Equus,* or for searching and sentinel duty, *e.g.,* a canine animal including domestic dogs and other members of the genus *Canis;* and domesticated quadrupeds being raised primarily for recreational purposes, *e.g.,* members of *Equus* and *Canis,* as well as a feline animal including domestic cats and other members of the family Felidae, genus *Felis.*

In addition to livestock animals and humans, the other most preferred species of animals for treatment with the nitric oxide releasing oxindole prodrugs of the present invention are the so-called "companion animals", which consist for the most part of cats and dogs. Dogs in particular are well known as being very susceptible to chronic inflammatory processes such as osteoarthritis and degenerative joint disease, which in dogs often results from a variety of developmental diseases, *e.g*., hip dysplasia and osteochondrosis, as well as from traumatic injuries to joints. Conventional NSAIDs, if used in canine therapy, have the potential for serious adverse gastrointestinal reactions and other adverse reactions including kidney and liver toxicity. Gastrointestinal effects such as single or multiple ulcerations, including perforation and hemorrhage of the esophagus, stomach, duodenum or small and large intestine, are usually debilitating, but can often be severe or even fatal.

Further included within the scope of the present invention are metabolites or residues of the compounds of Formula (I) which possess biological activity such that they are able to treat or prevent pain, fever, and inflammation, while at the same time they are able to release NO and thereby maintain gastric mucosal blood flow and prevent leukocyte adherence within the gastric microcirculation. In this manner, such metabolites or residues that release NO will be capable of counteracting the detrimental effects of cyclooxygenase suppression caused by the NSAID itself. Metabolites and residues of the compounds of Formula (I) also possess biological activity which allows them to modify an inflammatory disease or condition by favorably affecting its outcome through the prevention or diminution of those disease processes which cause tissue damage as a concomitant of inflammation. There is a disruption of the underlying mechanisms causing irreparable and irreversible tissue damage.

The novel compounds of the present invention not only modify the course of the inflammatory disease being treated, as demonstrated by accepted animal models of acute and chronic inflammation, but at the same time said novel compounds also produce a markedly favorable modification in the course of any concomitant gastric injury in the patient being treated, when administered on both an acute and a chronic basis. Indeed, the NO-releasing compounds of the present invention inhibit the release of reactive oxygen metabolites and proteases from white blood cells within the gastrointestinal microcirculation, and prevent a reduction in such circulation, thereby producing a significant acceleration in the healing of any gastric ulcers which may form.

Once synthesized, the inflammatory disease modifying as well as analgesic, antipyretic and anti-inflammatory activities and specificities of the compounds of Formula (I) may be determined using *in vitro* and *in vivo* assays which are well known in the art and are described in detail in technical publications available to the artisan. Further, once synthesized, the activities and specificities of the compounds of Formula (I) with regard to NO release, gastric mucosal blood flow maintenance, and leukocyte adherence prevention within the gastric microcirculation may be determined using *in vitro* and *in vivo* assays which are well known in the art and are described in detail in technical publications available to the artisan. In this way it is possible to assess the extent to which the compounds of Formula (I) are capable of counteracting the detrimental effects of cyclooxygenase suppression caused by the NSAID involved.

The desirable biological activity of the compounds of Formula (I) may also be improved by appending thereto appropriate functionalities which will function to enhance existing biological properties of the compound, improve the selectivity of the compound for the existing biological activities, or add to the existing biological activities further desirable biological activities. Such modifications are known in the art and include those which increase biological penetration into a given biological system, e.g., blood, the lymphatic system, and central nervous system; increase oral availability; increase solubility to allow administration by injection; alter metabolism; and alter the rate of excretion of the compound of Formula (I).

In view of the above definitions and others throughout the instant specification, other chemical and biological terms used herein can be easily understood by those of skill in the art The defined terms may be used alone or in any combination thereof. The preferred and more preferred chain lengths of the radicals which have been specified herein apply to all such combinations.

Further pursuant to the descriptions above of certain preferred subgeneric and more preferred subgeneric definitions of the compounds of Formula (I), there follows an enumeration of preferred and more preferred species in order to provide a further illustration of the present invention:
5-Nitrato-valeric acid-[6-chloro-5-fluoro-2-oxindole-1-carboxamide-3-(2-thienylmethyl)] ester, (*E*) and (*Z*) separate isomers;
5-S-nitroso-valeric acid-[6-chloro-5-fluoro-2-oxindole-1-carboxamide-3-(2-thienylmethyl)] ester;
3-S-nitroso-propylcarbamic acid-[6-chloro-5-fluoro-2-oxindole-1-carboxamide-3-(2-thienylmethyl)] ester;
2-(3,5-Difluorophenyl)-3-nitrato-propylcarbamic acid-[6-chloro-5-fluoro-2-oxindole-1-carboxamide-3-(2-thienylmethyl)]ester;
4-(3,5-Dimethoxyphenyl)-5-nitrato-valeric acid-[6-chloro-5-fluoro-2-oxindole-1-carboxamide-3-(2-thienylmethyl)] ester;
[6-Chloro-5-fluoro-2-oxindole-1-carboxamide-3-(2-thienylmethyl)oxy]formic acid 1-[4-(nitrato)]butyl ester;
[6-Chloro-5-fluoro-2-oxindole-1-carboxamide-3-(2-thienylmethyl)oxy]-S-thioformic acid 1-(2-cyclopropyl-3-nitrato)propyl ester;
[6-Chloro-5-fluoro-2-oxindole-1-carboxamide-3-(2-thienylmethyl)oxy]formic acid 1-[3-(1-*tert*-butyl-2-nitrato)ethyl]cyclopentyl ester;
[6-Chloro-5-fluoro-2-oxindole-1-carboxamide-3-(2-thienylmethyl)oxy]-S-thioformic acid (4-cyclohexyl-4-nitrato-2-propyl)propyl ester;
[6-Chloro-5-fluoro-2-oxindole-1-carboxamide-3-(2-thienylmethyl)oxy]-S-thioformic acid 2-[(2-methoxy)phenyl-3-nitrato]propyl ester;
[6-Chloro-5-fluoro-2-oxindole-1-carboxamide-3-(2-thienylmethyl)oxy]formic acid 2-[(3,6-difluorophenyl)-5-nitrato]pentyl ester.

The above-described compounds of the present invention may be utilized in the form of acids, esters, or other chemical classes of compounds to which the compounds described belong. It is also within the scope of the present invention to utilize those compounds in the form of pharmaceutically acceptable salts derived from various organic and inorganic acids and bases in accordance with procedures well known in the art. Such well-known pharmaceutically acceptable salts include, but are not limited to acetate, adipate, alginate, aspartate, benzoate, benzenesulfonate, besylate, bisulfate, butyrate, citrate, camphorate, camphorsulfonate, cyclopentanepropionate, digluconate, dodecysulfate, ethanesulfonate, fumarate, glucoheptanoate, gluconate, glycerophosphate, hemisuccinate, hemisulfate, heptanoate, hexanoate, hippurate, hydrochloride, hydrobromide, hydroiodide, 2-hydroxyethanesulfonate, isethionate, lactate, lactobionate, maleate, mandelate, methanesulfonate, 2-naphthalenesulfonate, nicotinate, nitrate, oxalate, oleate, pamoate, pectinate, persulfate, 3-phenylpropionate, phosphonate, picrate, pivalate, propionate, salicylate, sodium phosphate, stearate, succinate, sulfate, sulfosalicylate, tartrate, thiocyanate, thiomalate, tosylate, and undecanoate.

Base salts of the compounds of the present invention include, but are not limited to ammonium salts; alkali metal salts such as sodium and potassium; alkaline earth metal salts such as calcium and magnesium; salts with organic bases such as dicyclohexylamine, meglumine, N-methyl-D-glucamine, tris-(hydroxymethyl)-methylamine (tromethamine), and salts with amino acids such as arginine, lysine, *etc.* Compounds of the present invention which comprise basic nitrogen-containing groups may be quaternized with such agents as (C₁-C₄)alkyl halides, *e.g.,* methyl, ethyl, *iso*-propyl and *tert*-butyl chlorides, bromides and iodides; di(C₁-C₄)alkyl sulfate, *e.g.*, dimethyl, diethyl and diamyl sulfates; (C₁₀-C₁₈)alkyl halides, *e.g*., decyl, dodecyl, lauryl, myristyl and stearyl chlorides, bromides and iodides; and aryl-(C₁-C₄) alkyl halides, *e.g.,* benzyl chloride and phenethyl bromide. Such salts permit the preparation of both water-soluble and oil-soluble compounds of the present invention.

Among the above-recited pharmaceutical salts those which are preferred include, but are not limited to acetate, besylate, citrate, fumarate, gluconate, hemisuccinate, hippurate, hydrochloride, hydrobromide, isethionate, mandelate, meglumine, nitrate, oleate, phosphonate, pivalate, sodium phosphate, stearate, sulfate, sulfosalicylate, tartrate, thiomalate, tosylate, and tromethamine.

Multiple salts forms are included within the scope of the present invention where a compound of the present invention contains more than one group capable of forming such pharmaceutically acceptable salts. Examples of typical multiple salt forms include, but are not limited to bitartrate, diacetate, difumarate, dimeglumine, diphosphate, disodium, and trihydrochloride.

The pharmaceutical compositions of the present invention comprise any one or more of the above-described inhibitory compounds of the present invention, or a pharmaceutically acceptable salt thereof as also above-described, together with a pharmaceutically acceptable carrier in accordance with the properties and expected performance of such carriers which are well-known in the pertinent art.

The term "carrier" as used herein includes acceptable diluents, excipients, adjuvants, vehicles, solubilization aids, viscosity modifiers, preservatives and other agents well known to the artisan for providing favorable properties in the final pharmaceutical composition. In order to illustrate such carriers, there follows a brief survey of pharmaceutically acceptable carriers that may be used in the pharmaceutical compositions of the present invention, and thereafter a more detailed description of the various types of ingredients Typical carriers include but are by no means limited to, ion exchange compositions; alumina; aluminum stearate; lecithin; serum proteins, *e.g.,* human serum albumin; phosphates; glycine, sorbic acid; potassium sorbate; partial glyceride mixtures of saturated vegetable fatty acids; hydrogenated palm oils; water; salts or electrolytes, *e.g.,* prolamine sulfate, disodium hydrogen phosphate, potassium hydrogen phosphate, sodium chloride, and zinc salts; colloidal silica; magnesium trisilicate; polyvinyl pyrrolidone; cellulose-based substances; *e.g.,* sodium carboxymethylcellulose; polyethylene glycol; polyacrylates; waxes; polyethylene-polyoxypropylene-block polymers; and wool fat.

More particularly, the carriers used in the pharmaceutical compositions of the present invention comprise various classes and species of additives which are members independently selected from the groups consisting essentially of those recited in the following paragraphs.

Acidifying and alkalizing agents are added to obtain a desired or predetermined pH and comprise acidifying agents, *e.g.*, acetic acid, glacial acetic acid, malic acid, and propionic acid. Stronger acids such as hydrochloric acid, nitric acid and sulfuric acid may be used but are less preferred. Alkalizing agents include, *e.g.*, edetol, potassium carbonate, potassium hydroxide, sodium borate, sodium carbonate, and sodium hydroxide. Alkalizing agents which contain active amine groups, such as diethanolamine and trolamine, may also be used.

Aerosol propellants are required where the pharmaceutical composition is to be delivered as an aerosol under significant pressure. Such propellants include, *e.g.*, acceptable fluorochlorohydrocarbons such as dichlorodifluoromethane, dichlorotetrafluoroethane, and trichloromonofluoromethane; nitrogen; or a volatile hydrocarbon such as butane, propane, isobutane or mixtures thereof.

Antimicrobial agents including antibacterial, antifungal and antiprotozoal agents are added where the pharmaceutical composition is topically applied to areas of the skin which are likely to have suffered adverse conditions or sustained abrasions or cuts which expose the skin to infection by bacteria, fungi or protozoa. Antimicrobial agents include such compounds as benzyl alcohol, chlorobutanol, phenylethyl alcohol, phenylmercuric acetate, potassium sorbate, and sorbic acid. Antifungal agents include such compounds as benzoic acid, butylparaben, ethylparaben, methylparaben, propylparaben, and sodium benzoate.

Antimicrobial preservatives are added to the pharmaceutical compositions of the present invention in order to protect them against the growth of potentially harmful microorganisms, which usually invade the aqueous phase, but in some cases can also grow in the oil phase of a composition. Thus, preservatives with both aqueous and lipid solubility are desirable. Suitable antimicrobial preservatives include, *e.g.,* alkyl esters of *p*-hydroxybenzoic acid, propionate salts, phenoxyethanol, methylparaben sodium, propylparaben sodium, sodium dehydroacetate, benzalkonium chloride, benzethonium chloride, benzyl alcohol, hydantoin derivatives, quaternary ammonium compounds and cationic polymers, imidazolidinyl urea, diazolidinyl urea, and trisodium ethylenediamine tetracetate (EDTA). Preservatives are preferably employed in amounts ranging from about 0.01% to about 2.0% by weight of the total composition.

Antioxidants are added to protect all of the ingredients of the pharmaceutical composition from damage or degradation by oxidizing agents present in the composition itself or the use environment, *e.g.,* anoxomer, ascorbyl palmitate, butylated hydroxyanisole, butylated hydroxytoluene, hypophosphorous acid, potassium metabisulfite, propyl octyl and dodecyl gallate, sodium metabisulfite, sulfur dioxide, and tocopherols.

Buffering agents are used to maintain a desired pH of a composition once established, from the effects of outside agents and shifting equilibria of components of the composition. The buffering may be selected from among those familiar to the artisan skilled in the preparation of pharmaceutical compositions. *e.g.,* calcium acetate, potassium metaphosphate, potassium phosphate monobasic, and tartaric acid.

Chelating agents are used to help maintain the ionic strength of the pharmaceutical composition and bind to and effectively remove destructive compounds and metals, and include, *e.g*., edetate dipotassium, edetate disodium, and edetic acid.

Dermatologically active agents are added to the pharmaceutical compositions of the present invention where they are to be applied topically, and include, *e.g.,* wound healing agents such as peptide derivatives, yeast, panthenol, hexylresorcinol, phenol, tetracycline hydrochloride, lamin and kinetin; glucocorticosteroids for treating inflammation, *e.g.,* hydrocortisone, dexamethasone, betamethasone, triamcinolone, fluocinolone and methylprednisolone; retinoids for treating acne, psoriasis, cutaneous aging, and skin cancer, *e.g.*, retinol, tretinoin, isotretinoin, etretinate, acitretin, and arotinoid; immunosuppressive agents for treating inflammation, *e.g*., dapsone and sulfasalazine; mild antibacterial agents for treating mild acne and other skin infections, *e.g.*, resorcinol, salicylic acid, benzoyl peroxide, erythromycin-benzoyl peroxide, erythromycin, and clindamycin, and for treating impetigo, *e.g.,* mupirocin; antifungal agents for treating tinea corporis, tinea pedis, candidiasis and tinea versicolor, *e.g.,* griseofulvin, azoles such as miconazole, econazole, itraconazole, fluconazole, and ketoconazole, and allylamines such as naftifine and terfinafine; antiviral agents for treating cutaneous herpes simplex, herpes zoster, and chickenpox, *e.g.,* acyclovir, famciclovir, and valacyclovir; antihistamines for treating pruritis, atopic and contact dermatitis, *e.g.*, diphenhydramine, terfenadine, astemizole, loratadine, cetirizine, acrivastine, and temelastine; topical anesthetics for relieving pain, irritation and itching, *e.g.,* benzocaine, lidocaine, dibucaine, and pramoxine hydrochloride; topical analgesics for relieving pain and inflammation, *e.g.,* methyl salicylate, camphor, menthol, and resorcinol; topical antiseptics for preventing infection, *e.g.,* benzalkonium chloride and povidone-iodine; and vitamins and derivatives thereof such as tocopherol, tocopherol acetate, retinoic acid and retinol.

Dispersing and suspending agents are used as aids for the preparation of stable formulations and include, *e.g.*, poligeenan, povidone, and silicon dioxide.

Emollients are agents, preferably non-oily and water-soluble, which soften and soothe the skin, especially skin that has become dry because of excessive loss of water. Such agents are used with pharmaceutical compositions of the present invention which are intended for topical applications, and include,, *e.g.,* hydrocarbon oils and waxes, triglyceride esters, acetylated monoglycerides, methyl and other alkyl esters of C₁₀-C₂₀ fatty acids, C₁₀-C₂₀ fatty acids, C₁₀-C₂₀ fatty alcohols, lanolin and derivatives, polyhydric alcohol esters such as polyethylene glycol (200-600), polyoxyethylene sorbitan fatty acid esters, wax esters, phospholipids, and sterols; emulsifying agents used for preparing oil-in-water emulsions; excipients, *e.g.*, laurocapram and polyethylene glycol monomethyl ether; humectants, *e.g.,* sorbitol, glycerin and hyaluronic acid; ointment bases, *e.g.,* petrolatum, polyethylene glycol, lanolin, and poloxamer; penetration enhancers, *e.g.,* dimethyl isosorbide, diethyl-glycol-monoethylether, 1-dodecylazacycloheptan-2-one, and dimethylsulfoxide (DMSO); preservatives, *e.g.,* benzalkonium chloride, benzethonium chloride, alkyl esters of p-hydroxybenzoic acid, hydantoin derivatives, cetylpyridinium chloride, propylparaben, quaternary ammonium compounds such as potassium benzoate, and thimerosal; sequestering agents comprising cyclodextrins; solvents, *e.g.,* acetone, alcohol, amylene hydrate, butyl alcohol, corn oil, cottonseed oil, ethyl acetate, glycerin, hexylene glycol, isopropyl alcohol, isostearyl alcohol, methyl alcohol, methylene chloride, mineral oil, peanut oil, phosphoric acid, polyethylene glycol, polyoxypropylene 15 stearyl ether, propylene glycol, propylene glycol diacetate, sesame oil, and purified water; stabilizers, *e.g.,* calcium saccharate and thymol; surfactants, *e.g.*, lapyrium chloride; laureth 4, *i.e*., α-dodecyl-ω-hydroxy-poly(oxy-1,2-ethanediyl) or polyethylene glycol monododecyl ether.

Emulsifying agents, including emulsifying and stiffening agents and emulsion adjuncts, are used for preparing oil-in-water emulsions when these form the basis of the pharmaceutical compositions of the present invention. Such emulsifying agents include, *e.g.,* non-ionic emulsifiers such as C₁₀-C₂₀ fatty alcohols and said fatty alcohols condensed with from 2 to 20 moles of ethylene oxide or propylene oxide, (C₆-C₁₂)alkyl phenols condensed with from 2 to 20 moles of ethylene oxide, mono- and di- C₁₀-C₂₀ fatty acid esters of ethylene glycol, C₁₀-C₂₀ fatty acid monoglyceride, diethylene glycol, polyethylene glycols of MW 200-6000, polypropylene glycols of MW 200-3000, and particularly sorbitol, sorbitan, polyoxyethylene sorbitol, polyoxyethylene sorbitan, hydrophilic wax esters, cetostearyl alcohol, oleyl alcohol, lanolin alcohols, cholesterol, mono- and di-glycerides, glyceryl monostearate, polyethylene glycol monostearate, mixed mono- and distearic esters of ethylene glycol and polyoxyethylene glycol, propylene glycol monostearate, and hydroxypropyl cellulose. Emulsifying agents which contain active amine groups may also be used and typically include anionic emulsifiers such as fatty acid soaps, *e.g.*, sodium, potassium and triethanolamine soaps of C₁₀-C₂₀ fatty acids; alkali metal, ammonium or substituted ammonium (C₁₀-C₃₀)alkyl sulfates, (C₁₀-C₃₀)alkyl sulfonates, and (C₁₀-C₅₀)alkyl ethoxy ether sulfonates. Other suitable emulsifying agents include castor oil and hydrogenated castor oil; lecithin; and polymers of 2-propenoic acid together with polymers of acrylic acid, both cross-linked with allyl ethers of sucrose and/or pentaerythritol, having varying viscosities and identified by product names carbomer 910, 934, 934P, 940, 941, and 1342. Cationic emulsifiers having active amine groups may also be used, including those based on quaternary ammonium, morpholinium and pyridinium compounds. Similarly, amphoteric emulsifiers having active amine groups, such as cocobetaines, lauryl dimethylamine oxide and cocoylimidazoline, may be used. Useful emulsifying and stiffening agents also include cetyl alcohol and sodium stearate; and emulsion adjuncts such as oleic acid, stearic acid, and stearyl alcohol.

Excipients include, *e.g.*, laurocapram and polyethylene glycol monomethyl ether.

Where the pharmaceutical composition of the present invention is to be applied topically, penetration enhancers may be used, which include, *e.g.,* dimethyl isosorbide, diethyl-glycol-monoethylether, 1-dodecylazacycloheptan-2-one, and dimethylsulfoxide (DMSO). Such compositions will also typically include ointment bases, *e.g*., petrolatum, polyethylene glycol, lanolin, and poloxamer, which is a block copolymer of polyoxyethylene and polyoxypropylene, which may also serve as a surfactant or emulsifying agent, and which may be represented by Formula (XVI):

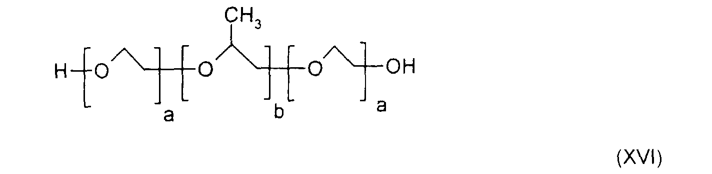

Preservatives are used to protect pharmaceutical compositions of the present invention from degradative attack by ambient microorganisms, and include, eg, benzalkonium chloride, benzethonium chloride, alkyl esters of p-hydroxybenzoic acid, hydantoin derivatives, cetylpyridinium chloride, monothioglycerol, phenol, phenoxyethanol, methylparagen, imidazolidinyl urea, sodium dehydroacetate, propylparaben, quaternary ammonium compounds, especially polymers such as polixetonium chloride, potassium benzoate, sodium formaldehyde sulfoxylate, sodium propionate, and thimerosal.

Sequestering agents are used to improve the stability of the pharmaceutical compositions of the present invention and include, *e.g.,* the cyclodextrins which are a family of natural cyclic oligosaccharides capable of forming inclusion complexes with a variety of materials, and are of varying ring sizes, those having 6-, 7- and 8-glucose residues in a ring being commonly referred to as α-cyclodextrins, β-cyclodextrins, and γ-cyclodextrins, respectively. Suitable cyclodextrins include, *e.g.,* α-cyclodextrin, β-cyclodextrin, γ-cyclodextrin, δ-cyclodextrin and cationized cyclodextrins.

Solvents which may be used in preparing the pharmaceutical compositions of the present invention include, *e.g.,* acetone, alcohol, amylene hydrate, butyl alcohol, corn oil, cottonseed oil, ethyl acetate, glycerin, hexylene glycol, isopropyl alcohol, isostearyl alcohol, methyl alcohol, methylene chloride, mineral oil, peanut oil, phosphoric acid, polyethylene glycol, polyoxypropylene 15 stearyl ether, propylene glycol, propylene glycol diacetate, sesame oil, and purified water.

Stabilizers which are suitable for use include, *e.g.*, calcium saccharate and thymol.

Stiffening agents are typically used in formulations for topical applications in order to provide desired viscosity and handling characteristics and include, *e.g.,* cetyl esters wax, myristyl alcohol, parafin, synthetic parafin, emulsifying wax, microcrystalline wax, white wax and yellow wax.

Sugars are often used to impart a variety of desired characteristics to the pharmaceutical compositions of the present invention and in order to improve the results obtained, and include, *e.g.,* monosaccharides, disaccharides and polysaccharides such as glucose, xylose, fructose, reose, ribose, pentose, arabinose, allose, tallose, altrose, mannose, galactose, lactose, sucrose, erythrose, glyceraldehyde, or any combination thereof.

Surfactants are employed to provide stability for multi-component pharmaceutical compositions of the present invention, enhance existing properties of those compositions, and bestow desirable new characteristics on said compositions. Surfactants are used as wetting agents, antifoam agents, for reducing the surface tension of water, and as emulsifiers, dispersing agents and penetrants, and include, *e.g.*, lapyrium chloride; laureth 4, *i.e*., α-dodecyl-ω-hydroxy-poly(oxy-1,2-ethanediyl) or polyethylene glycol monododecyl ether; laureth 9, *i.e*., a mixture of polyethylene glycol monododecyl ethers averaging about 9 ethylene oxide groups per molecule; monoethanolamine; nonoxynol 4, 9 and 10, *i.e.,* polyethylene glycol mono(p-nonylphenyl) ether; nonoxynol 15, *i.e.,* α-(*p*-nonylphenyl)-ω-hydroxypenta-deca(oxyethylene); nonoxynol 30, *i.e.,* α-(*p*-nonylphenyl)-ω-hydroxytriaconta(oxyethylene); poloxalene, *i.e.,* nonionic polymer of the polyethylene-polypropylene glycol type, MW = approx. 3000; poloxamer, referred to in the discussion of ointment bases further above; polyoxyl 8, 40 and 50 stearate, *i.e*., poly(oxy-1,2-ethanediyl), α-hydro-ω-hydroxy-; octadecanoate; polyoxyl 10 oleyl ether, *i.e*., poly(oxy-1,2-ethanediyl), α-[(Z)-9-octadecenyl-ω-hydroxy-; polysorbate 20, *i.e.,* sorbitan, monododecanoate, poly(oxy-1,2-ethanediyl); polysorbate 40, *i.e.,* sorbitan, monohexadecanoate, poly(oxy-1,2-ethanediyl); polysorbate 60, *i.e.,* sorbitan, monooctadecanoate, poly(oxy-1,2-ethanediyl); polysorbate 65, *i.e.,* sorbitan, trioctadecanoate, poly(oxy-1,2-ethanediyl); polysorbate 80, *i.e.,* sorbitan, mono-9-monodecenoate, poly(oxy-1,2-ethanediyl); polysorbate 85, *i.e.,* sorbitan, tri-9-octadecenoate, poly(oxy-1,2-ethanediyl); sodium lauryl sulfate; sorbitan monolaurate; sorbitan monooleate; sorbitan monopalmitate; sorbitan monostearate; sorbitan sesquioleate; sorbitan trioleate; and sorbitan tristearate. A further description of additional types of auxiliary ingredients which may optionally be included in the pharmaceutical compositions of the present invention, as well as of specific compositions of those types and of the above-described types which may thus be optionally included, may be found in the *CTFA International Cosmetic Ingredient Dictionary,* 4th ed., The Cosmetic, Toiletry, and Fragrance Association, Inc., Washington, D.C., 1991, which is incorporated herein with respect to the portions thereof relevant to the pharmaceutical compositions of the present invention.

Lubricity, the property of having a smooth or slippery quality, is a desirable property of the pharmaceutical compositions of the present invention which are to be applied topically. Lubricity can be enhanced by the addition of agents well known in the art for that purpose, which include, *e.g.,* a silicone oil or fluid which is preferred since it can provide the desired lubricity and emollience without a greasy feel. Other lubricity agents include dimethyl polysiloxane and methylphenyl polysiloxane which are water-soluble, and silicone glycol copolymer which is alcohol-soluble. Polysiloxanes which are commonly employed include dimethyl polysiloxane which is end-blocked with trimethyl units, the CTFA name for which is dimethicone, and polydimethylcyclosiloxane, the CTFA name for which is cyclomethicone. Such preferred siloxanes exhibit a viscosity of from about 2 to about 50 centistokes at 25° C.

The pharmaceutical compositions of the present invention may be prepared using very straightforward methodology which is well understood by the artisan of ordinary skill. Where the pharmaceutical compositions of the present invention are simple aqueous and/or other solvent solutions, the various components of the overall composition are brought together in any practical order, which will be dictated largely by considerations of convenience. Those components having reduced water solubility, but sufficient solubility in the same co-solvent with water, may all be dissolved in said co-solvent, after which the co-solvent solution will be added to the water portion of the carrier whereupon the solutes therein will become dissolved in the water. To aid in this dispersion/solution process, a surfactant may be employed.

Where the pharmaceutical compositions of the present invention are to be in the form of emulsions, the components of the pharmaceutical composition will be brought together in accordance with the following general procedures. The continuous water phase is first heated to a temperature in the range of from about 60° to about 95°C, preferably from about 70° to about 85°C, the choice of which temperature to use being dependent upon the physical and chemical properties of the components which make up the oil-in-water emulsion. Once the continuous water phase has reached its selected temperature, the components of the final composition to be added at this stage are admixed with the water and dispersed therein under high-speed agitation. Next, the temperature of the water is restored to approximately its original level, after which the components of the composition which comprise the next stage are added to the composition mixture under moderate agitation and mixing continues for from about 5 to about 60 minutes, preferably about 10 to about 30 minutes, depending on the components of the first two stages. Thereafter, the composition mixture is passively or actively cooled to from about 20° to about 55°C for addition of any components in the remaining stages, after which water is added in sufficient quantity to reach its original predetermined concentration in the overall composition.

According to this invention, the pharmaceutical compositions may be in the form of a sterile injectable preparation, for example a sterile injectable aqueous or oleaginous suspension. This suspension may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as do natural pharmaceutically-acceptable oils, such as olive oil or castor oil, especially in their polyoxyethylated versions. These oil solutions or suspensions may also contain a long-chain alcohol diluent or dispersant, such as *Rh,* HCIX or similar alcohol.

The pharmaceutical compositions of this invention may be orally administered in any orally acceptable dosage form including, but not limited to, capsules, tablets, aqueous suspensions or solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the anti-inflammatory active ingredient is combined with emulsifying and suspending agents. If desired, certain sweetening, flavoring or coloring agents may also be added. Alternatively, the pharmaceutical compositions of this invention may be administered in the form of suppositories for rectal administration. These can be prepared by mixing the agent with a suitable non-irritating excipient which is solid at room temperature but liquid at the rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation, as described above, or in a suitable enema formulation. Topically active transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate, cetyl esters wax, cetearyl alcohol, 2-octyldodecanol, benzyl alcohol and water.

Pharmaceutical compositions within the scope of the present invention include those wherein the therapeutically effective amount for treating pain and inflammation of a nitric oxide releasing oxindole prodrug as described herein is provided in a dosage form suitable for systemic administration. Such a pharmaceutical composition will contain the anti-inflammatory active ingredient in suitable liquid form for delivery by: (1) injection or infusion which is intraarterial, intra- or transdermal, subcutaneous, intramuscular, intraspinal, intrathecal, or intravenous, wherein said anti-inflammatory active ingredient: (a) is contained in solution as a solute; (b) is contained in the discontinuous phase of an emulsion, or the discontinuous phase of an inverse emulsion which inverts upon injection or infusion, said emulsions containing suitable emulsifying agents; or (c) is contained in a suspension as a suspended solid in colloidal or microparticulate form, said suspension containing suitable suspending agents; (2) injection or infusion into suitable body tissues or cavities as a depot, wherein said composition provides storage of said anti-inflammatory active ingredient and thereafter delayed-, sustained-, and/or controlled-release of said anti-inflammatory active ingredient for systemic distribution; (3) instillation, inhalation or insufflation into suitable body tissues or cavities of said pharmaceutical composition in suitable solid form, where said anti-inflammatory active ingredient: (a) is contained in a solid implant composition providing delayed-, sustained-, and/or controlled-release of said inhibitor; (b) is contained in a particulate composition to be inhaled into the lungs; or (c) is contained in a particulate composition to be blown into suitable body tissues or cavities, where said composition optionally provides delayed-, sustained-, and/or controlled-release of said inhibitor; or (4) ingestion of said pharmaceutical composition in suitable solid or liquid form for peroral delivery of said inhibitor, where said inhibitor: (a) is contained in a solid dosage form; or (b) is contained in a liquid dosage form.

Particular dosage forms of the above-described pharmaceutical compositions include (1) suppositories as a special type of implant, comprising bases which are solid at room temperature but melt at body temperature, slowly releasing the anti-inflammatory active ingredient with which they are impregnated into the surrounding tissue of the body, where the anti-inflammatory active ingredient becomes absorbed and transported to effect systemic administration; (2) solid peroral dosage forms selected from the group consisting of (a) delayed-release oral tablets, capsules, caplets, lozenges, troches, and multiparticulates, (b) enteric-coated tablets and capsules which prevent release and absorption in the stomach to facilitate delivery distal to the stomach of the patient being treated, (c) sustained-release oral tablets, capsules and microparticulates which provide systemic delivery of the anti-inflammatory active ingredient in a controlled manner up to a 24-hour period, (d) fast-dissolving tablets, (e) encapsulated solutions, (f) an oral paste, (g) a granular form incorporated in or to be incorporated in the food of a livestock animal or a livestock animal or a companion animal which is being treated, (h) a chewable form in which said anti-inflammatory active ingredient is consumed along with the palatable chew by a livestock animal or a companion animal being treated, or may alternatively be delivered by leaching from the body of the chew which is not consumed, during mastication by a livestock animal or a companion animal being treated; and (i) liquid peroral dosage forms selected from the group consisting of solutions, suspensions, emulsions, inverse emulsions, elixirs, extracts, tinctures, and concentrates, optionally to be added to the drinking water of a livestock animal or a companion animal being treated.

Pharmaceutical compositions within the scope of the present invention include those wherein the therapeutically effective amount for treating pain and inflammation of a nitric oxide releasing oxindole prodrug as described herein is provided in a dosage form suitable for local administration to a site of inflammation in an animal being treated, wherein said pharmaceutical composition contains said anti-inflammatory active ingredient in suitable liquid form for delivering said anti-inflammatory active ingredient by: (1) injection or infusion into a local site of inflammation, which is intraarterial, intraarticular, intrachondrial, intracostal, intracystic, intra- or transdermal, intrafasicular, intraligamentous, intramedulary, intramuscular, intranasal, intraneural, intraocular, *i.e.,* opthalmic administration, intraosteal, intrapelvic, intrapericardial, intraspinal, intrasternal, intrasynovial, intratarsal, or intrathecal; including components which provide delayed-release, controlled-release, and/or sustained-release of said anti-inflammatory active ingredient into said local site of inflammation; where said anti-inflammatory active ingredient is contained: (a) in solution as a solute; (b) in the discontinuous phase of an emulsion, or the discontinuous phase of an inverse emulsion which inverts upon injection or infusion, said emulsions containing suitable emulsifying agents; or (c) in a suspension as a suspended solid in colloidal or microparticulate form, said suspension containing suitable suspending agents; or (2) injection or infusion as a depot for delivering said anti-inflammatory active ingredient to said local site of inflammation; wherein said composition provides storage of said anti-inflammatory active ingredient and thereafter delayed-, sustained-, and/or controlled-release of said anti-inflammatory active ingredient into said local site of inflammation, and wherein said composition also includes components which ensure that said inhibitor has predominantly local activity, with little systemic carryover activity; or wherein said pharmaceutical composition contains said anti-inflammatory active ingredient in suitable solid form for delivering said inhibitor by: (3) instillation, inhalation or insufflation to said local site of inflammation, where said anti-inflammatory active ingredient is contained: (a) in a solid implant composition which is installed in said local site of inflammation, said composition optionally providing delayed-, sustained-, and/or controlled-release of said anti-inflammatory active ingredient to said local site of inflammation; (b) in a particulate composition which is inhaled into a local site of inflammation comprising the lungs; or (c) in a particulate composition which is blown into a local site of inflammation, where said composition includes components which will ensure that said anti-inflammatory active ingredient has predominantly local activity, with insignificant systemic carryover activity, and optionally provides delayed-, sustained-, and/or controlled-release of said anti-inflammatory active ingredient to said local site of inflammation. For ophthalmic use, the pharmaceutical compositions may be formulated as micronized suspension in isotonic, pH adjusted sterile saline, or, preferably, as solutions in isotonic, pH adjusted sterile saline, either with our without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation through the use of a nebulizer, a dry powder inhaler or a metered dose inhaler. Such compositions are prepared according to techniques well-known in the art of pharmaceutical formulation and may be prepared as solutions in saline, employing benzyl alcohol or other suitable preservatives, absorption promoters to enhance bioavailability, hydrofluorocarbons, and/or other conventional solubilizing or dispersing agents.

As already mentioned, the anti-inflammatory active ingredients of Formula (I) of the present invention may be administered systemically to a patient to be treated as a pharmaceutical composition in suitable liquid form by injection or infusion. There are a number of sites and organ systems in the body of the patient which will allow the properly formulated pharmaceutical composition, once injected or infused, to permeate the entire body and all of the organ system of the patient being treated. An injection is a single dose of the pharmaceutical composition forced, usually by a syringe, into the tissue involved. The most common types of injections are intramuscular, intravenous, and subcutaneous. By contrast, an infusion is the gradual introduction of the pharmaceutical composition into the tissue involved. The most common type of infusion is intravenous. Other types of injection or infusion comprise intraarterial, intra- or transdermal (including subcutaneous), or intraspinal especially intrathecal. In these liquid pharmaceutical compositions, the anti-inflammatory active ingredient may be contained in solution as the solute. This is the most common and most preferred type of such composition, but requires an anti-inflammatory active ingredient in a salt form that has reasonably good aqueous solubility. Water (or saline) is by far the most preferred solvent for such compositions. Occasionally supersaturated solutions may be utilized, but these present stability problems that make them impractical for use on an everyday basis.

If it is not possible to obtain a form of some compound of Formula (I) that has the requisite degree of aqueous solubility, as may sometimes occur, it is within the skill of the artisan to prepare an emulsion, which is a dispersion of small globules of one liquid, the discontinuous or internal phase, throughout a second liquid, the continuous or external phase, with which it is immiscible. The two liquids are maintained in an emulsified state by the use of emulsifiers which are pharmaceutically acceptable. Thus, if the anti-inflammatory active ingredient is a water-insoluble oil, it can be administered in an emulsion of which it is the discontinuous phase. Also where the active ingredient is water-insoluble but can be dissolved in a solvent which is immiscible with water, an emulsion can be used. While the active ingredient would most commonly be used as the discontinuous or internal phase of what is referred to as an oil-in-water emulsion, it could also be used as the discontinuous or internal phase of an inverse emulsion, which is commonly referred to as a water-in-oil emulsion. Here the anti-inflammatory active ingredient is soluble in water and could be administered as a simple aqueous solution. However, inverse emulsions invert upon injection or infusion into an aqueous medium such as the blood, and offer the advantage of providing a more rapid and efficient dispersion of the anti-inflammatory active ingredient into that aqueous medium than can be obtained using an aqueous solution. Inverse emulsions are prepared by using suitable, pharmaceutically acceptable emulsifying agents well known in the art. Where the anti-inflammatory active ingredient has limited water solubility, it may also be administered as a suspended solid in colloidal or microparticulate form in a suspension prepared using suitable, pharmaceutically acceptable suspending agents. The suspended solids containing the anti-inflammatory active ingredient may also be formulated as delayed-, sustained-, and/or controlled-release compositions.

While systemic administration will most frequently be carried out by injection or infusion of a liquid, there are many situations in which it will be advantageous or even necessary to deliver the anti-inflammatory active ingredient as a solid. Systemic administration of solids is carried out by instillation, inhalation or insufflation of a pharmaceutical composition in suitable solid form containing the anti-inflammatory active ingredient. Instillation of the anti-inflammatory active ingredient may entail installing a solid implant composition into suitable body tissues or cavities. The implant may comprise a matrix of bio-compatible and bio-erodible materials in which particles of a solid anti-inflammatory active ingredient are dispersed, or in which, possibly, globules or isolated cells of a liquid anti-inflammatory active ingredient are entrapped. Desirably, the matrix will be broken down and completely absorbed by the body. The composition of the matrix is also preferably selected to provide controlled-, sustained-, and/or delayed release of the anti-inflammatory active ingredient over extended periods of time, even as much as several months.

The term "implant" most often denotes a solid pharmaceutical composition containing the anti-inflammatory active ingredient, while the term "depot" usually implies a liquid pharmaceutical composition containing the anti-inflammatory active ingredient, which is deposited in any suitable body tissues or cavities to form a reservoir or pool which slowly migrates to surrounding tissues and organs and eventually becomes systemically distributed. However, these distinctions are not always rigidly adhered to in the art, and consequently, it is contemplated that there is included within the scope of the present invention liquid implants and solid depots, and even mixed solid and liquid forms for each. Suppositories may be regarded as a type of implant, since they comprise bases which are solid at room temperature but melt at a patient's body temperature, slowly releasing the anti-inflammatory active ingredient with which they are impregnated into the surrounding tissue of the patient's body, where the anti-inflammatory active ingredient becomes absorbed and transported to effect systemic administration.

Systemic administration can also be accomplished by inhalation or insufflation of a powder, *i.e.,* particulate composition containing the anti-inflammatory active ingredient For example, the anti-inflammatory active ingredient in powder form may be inhaled into the lungs using conventional devices for aerosolizing particulate formulations. The anti-inflammatory active ingredient as a particulate formulation may also be administered by insufflation, *i.e.,* blown or otherwise dispersed into suitable body tissues or cavities by simple dusting or using conventional devices for aerosolizing particulate formulations. These particulate compositions may also be formulated to provide delayed-, sustained-, and/or controlled-release of the anti-inflammatory active ingredient in accordance with well understood principles and known materials.

Other means of systemic administration which may utilize the anti-inflammatory active ingredients of the present invention in either liquid or solid form include transdermal, intranasal, and opthalmic routes. In particular, transdermal patches prepared in accordance with well known drug delivery technology may be prepared and applied to the skin of a patient to be treated, whereafter the active agent by reason of its formulated solubility characteristics migrates across the epidermis and into the dermal layers of the patient's skin where it is taken up as part of the general circulation of the patient, ultimately providing systemic distribution of the anti-inflammatory active ingredient over a desired, extended period of time. Also included are implants which are placed beneath the epidermal layer of the skin, *i.e.* between the epidermis and the dermis of the skin of the patient being treated. Such an implant will be formulated in accordance with well known principles and materials commonly used in this delivery technology, and may be prepared in such a way as to provide controlled-, sustained-, and/or delayed-release of the anti-inflammatory active ingredient into the systemic circulation of the patient. Such subepidermal (subcuticular) implants provide the same facility of installation and delivery efficiency as transdermal patches, but without the limitation of being subject to degradation, damage or accidental removal as a consequence of being exposed on the top layer of the patient's skin.

The amount of anti-inflammatory active ingredient that may be combined with the carrier materials to produce a single dosage form will vary depending upon the host treated, and the particular mode of administration. It should be understood, however, that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of anti-inflammatory active ingredient may also depend upon the therapeutic or prophylactic agent, if any, with which the ingredient is co-administered

The dosage and dose rate of the compounds of Formula (I) effective for treating or preventing pain, inflammation, fever, and gastrointestinal lesions in a patient, as well as for modifying an osteoarthritic, inflammatory, or degenerative joint disease or condition by favorably affecting the outcome thereof in said patient, usually a mammal in need of such treatment, in accordance with the method of the present invention comprising administering to said patient usually a mammal a therapeutically effective amount of a compound of Formula (I) will depend on a variety of factors, such as the nature of the anti-inflammatory active ingredient, the size of the patient, the goal of the treatment, the nature of the pathology to be treated, the specific pharmaceutical composition used, and the observations and conclusions of the treating physician.

Generally, however, the effective therapeutic dose of a compound of Formula (I) which will be administered to a patient will be between about 10 µg (0.01 mg)/kg and about 10.0 mg/kg of body weight per day, preferably between about 100 µg (0.1 mg)/kg and about 8.0 mg/kg of body weight per day, more preferably between about 1.0 mg/kg and about 6.0 mg/kg of body weight per day, and most preferably between about 2.0 mg/kg and about 4.0 mg/kg of body weight per day of the anti-inflammatory active ingredient of Formula (I).

Where the dosage form is oral, *e.g.,* a tablet or capsule, suitable dosage levels of the compounds of Formula (I) will be between about 100 µg (0.1 mg)/kg and about 5.0 mg/kg body weight per day, preferably between about 1.0 mg/kg and about 4.5 mg/kg body weight per day, more preferably between about 1.5 mg/kg and about 4.0 mg/kg of body weight per day, and most preferably between about 2.0 mg/kg and about 3.5 mg/kg of body weight per day of the anti-inflammatory active ingredient of Formula (I).

Where the dosage form is topically administered to the bronchia and lungs, *e.g.,* by means of a powder inhaler or nebulizer, suitable dosage levels of the compounds of Formula (I) will be between about 100 µg (0.1 mg)/kg and about 3.5 mg/kg body weight per day, preferably between about 500 µg (0.5 mg)/kg and about 3.0 mg/kg body weight per day, more preferably between about 1.0 mg and about 2.5 mg/kg of body weight per day, and most preferably between about 1.5 mg/kg and about 2.0 mg/kg of body weight per day of the anti-inflammatory active ingredient of Formula (I).

Using representative body weights of 10 kg and 100 kg in order to illustrate the range of daily topical dosages which might be used as described above, suitable dosage levels of the compounds of Formula (I) will be between about 1.0 -10.0 µg and 10.0 - 100.0 mg per day, preferably between about 5.0-50.0 µg and 5.0-50.0 mg per day, more preferably between about 10.0 - 100.0 µg and 1.0 - 10.0 mg per day, and most preferably between about 20.0 -200.0 µg and about 0.5 - 5.0 mg per day of the anti-inflammatory active ingredient comprising a compound of Formula (I). These ranges of dosage amounts represent total dosage amounts of the anti-inflammatory active ingredient per day for a given patient. The number of times per day that a dose is administered will depend upon such pharmacological and pharmacokinetic factors as the half-life of the anti-inflammatory active ingredient, which reflects its rate of catabolism and clearance, as well as the minimal and optimal blood plasma or other body fluid levels of said anti-inflammatory active ingredient attained in the patient which are required for therapeutic efficacy

Numerous other factors must also be considered in deciding upon the number of doses per day and the amount of anti-inflammatory active ingredient per dose which will be administered. Not the least important of such other factors is the individual response of the patient being treated. Thus, for example, where the anti-inflammatory active ingredient is used to treat or prevent asthma, and is administered topically *via* aerosol inhalation into the lungs, from one to four doses consisting of acuations of a dispensing device, *i.e.,* "puffs" of an inhaler, will be administered each day, each dose containing from about 50.0 µg to about 10 0 mg of anti-inflammatory active ingredient.

Included within the scope of the present invention are embodiments comprising compositions which contain, in addition to a compound of the present invention as anti-inflammatory active ingredient, additional therapeutic agent anti-inflammatory active ingredients such as anti-inflammatory corticosteroids; bronchodilators; anti-asthmatics; other non-steroidal anti-inflammatories; and immunostimulants. Specific compounds within such classes may be selected from those listed under the appropriate headings in *Comprehensive Medicinal Chemistry,* Pergamon Press, Oxford, England, pp. 970-986 (1990); and *Goodman and Gilman's The Pharmacological Basis of Therapeutics,* 9th ed., Hardman, J. G. and Limbird, L. E., eds., McGraw-Hill, 1996, the disclosure of which are incorporated herein by reference in their entireties. In those cases where a joint has become seriously inflammed and infected at the same time by microorganisms, *e.g.,* bacteria, fungi, protozoa, virus and the like, the anti-inflammatory active ingredient of the present invention will desirably be administered in combination with one or more antibiotic, antifungal, antiprotozoal, antiviral or similar therapeutic agents. Further, the anti-inflammatory active ingredient of the present invention may be administered not only in combination with other NSAIDs as already indicated, but in combination as well with inhibitors of other mediators of inflammation, comprising one or more members selected from the group consisting essentially of the classes of such inhibitors and examples thereof which include, H₁-receptor antagonists; kinin-B₁ - and B₂ -receptor antagonists; prostaglandin inhibitors such as PGD-, PGF- PGI₂ -, and PGE-receptor antagonists; thromboxane A₂ (TXA2-) inhibitors; 5- and 12-lipoxygenase inhibitors; leukotriene LTC₄ -, LTD₄/LTE₄ -, and LTB₄ -inhibitors; PAF-receptor antagonists; gold in the form of an aurothio group together with various hydrophilic groups; immunosuppressive agents, *e.g*., cyclosporine, azathioprine, and methotrexate; anti-inflammatory glucocorticoids; penicillamine; hydroxychloroquine; anti-gout agents, *e.g.,* colchicine, xanthine oxidase inhibitors, *e.g.,* allopurinol, and uricosuric agents, *e.g.,* probenecid, sulfinpyrazone, and benzbromarone. Other examples include anti-inflammatory compounds such as theophylline, sulfasalazine and aminosalicylates; and immunomodulators such as the interferons.

Still further embodiments of the present invention relate to a method of treating or preventing an inflammatory disease or condition, especially a method of treating or preventing pain, inflammation, fever, and gastrointestinal lesions in a patient in need of such treatment, comprising administering to said patient a therapeutically effective amount of a compound of Formula (I). There is further provided a non-enteropathogenic method of treating an inflammatory disease or condition in a patient in need of such treatment, comprising administering to said patient a therapeutically effective amount of a compound of Formula (I).

The above-described methods of treatment of the present invention may employ the compounds of Formula (I) in the form of monotherapy, but said methods may also be used in the form of multiple therapy in which one or more compounds of Formula (I) are coadministered in combination with one or more known therapeutic agents such as those described in detail further above. The terms "coadministered" or "coadministration" as used herein are intended to mean therapeutic utilization of one or more compounds of Formula (I) in combination with one or more additional therapeutic agents, including but not limited to administration of the combination of therapeutic active agents in a single dosage form or in multiple dosage forms representing the same or different routes of administration, said multiple dosage forms being administered at substantially the same time or at different times.

The compounds of the present invention may be formulated into pharmaceutical compositions that may be administered orally, parenterally, by inhalation (metered dose inhaler, dry powder inhaler or nebulizer), topically, rectally, nasally, intraocularly, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques.

The compounds of Formula (I) may be prepared in accordance with well-known procedures for carrying out the synthesis of organic compounds which comprise several heterocyclic ring systems. A number of different procedures are available which are fully disclosed in the technical literature and with which the skilled artisan will be familiar. The description which follows of one such synthesis scheme is merely representative and is not intended to be in any way limiting with respect to the scope of the present invention.

The following schematic synthesis diagram illustrates a generalized preparation process for the compound of Formula (I) which is 5-nitrato-valeric acid-[6-chloro-5-fluoro-2-oxindole-1-carboxamide-3-(2-thienylmethyl)] ester, including a mixture of the (*E*) and (*Z*) isomers which are then separated.

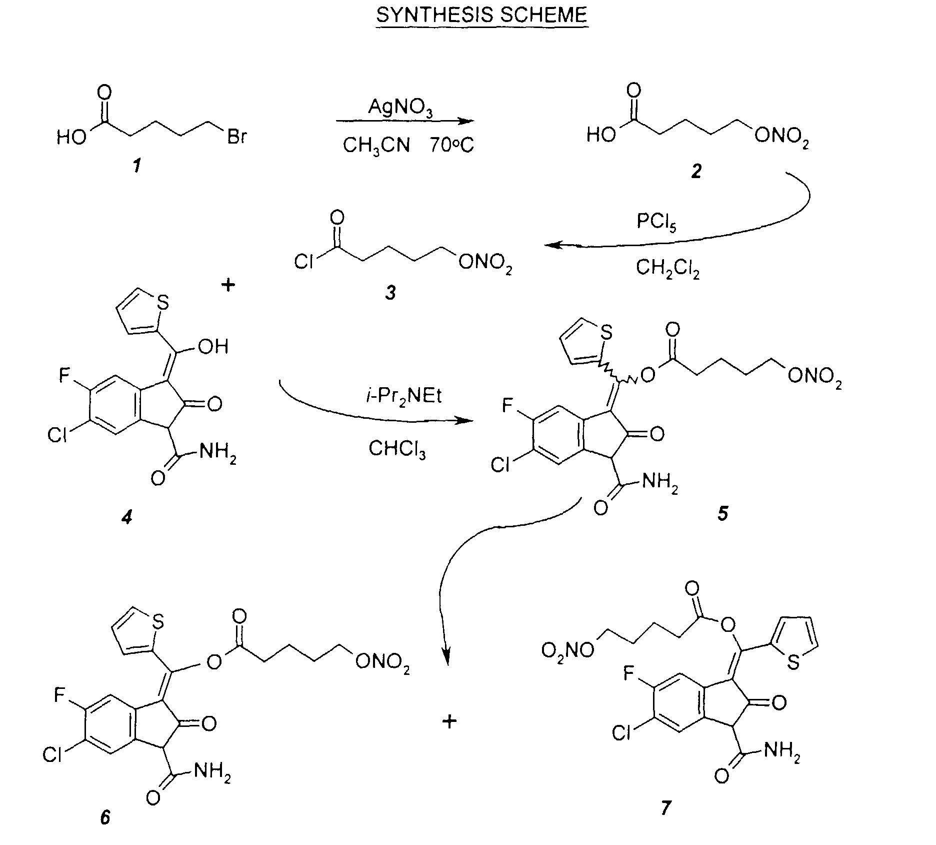

### DESCRIPTION OF PREFERRED EMBODIMENTS

The following examples are presented in order to further illustrate the novel compositions of matter, methods of treatment and pharmaceutical compositions of the present invention, but are not intended to in any way be taken as limiting the scope of the present invention. Only the claims attached hereto serve to point out and thereby limit the scope of the present invention.

### EXAMPLE 1

### Nitric Oxide Releasing Oxindole Prodrugs with Analgesic, Anti-inflammatory, and Disease-Modifying Activity

### A. 5-Nitrato-valeric acid

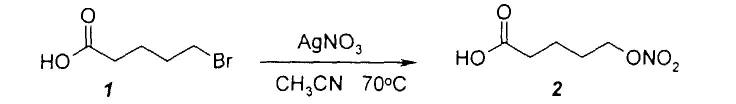

Silver nitrate (170 g, 1.00 mol, 5, equiv.) was dissolved in 500 mL acetonitrile. The solution was warmed to 50°C with stirring under a nitrogen atmosphere. 5-Bromovaleric acid (36.2 g, 0.200 mol) was dissolved in 10 mL of acetonitrile and then added quickly to the silver nitrate solution *via* an addition funnel. A precipitate formed upon said addition, after which the reaction mixture was heated to 70°C and stirred for 20 minutes. After cooling, the silver bromide was removed by filtration and the resulting acetonitrile solution was concentrated. The product residue was then partitioned between EtOAc and water. The aqueous layer was extracted with more EtOAc and then the combined organic solutions were dried over sodium sulfate, filtered and concentrated. The residue was finally taken up in 1 L of ether to filter again through Whatman glass microfiber paper and then evaporated to provide 5-nitrato-valeric acid as a light orange oil (27.1 g, 0.166 mol, 83%). The acid was converted to the acid chloride without further purification.

### B. 5-Nitrato-valeric acid chloride

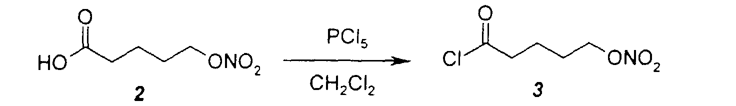

5-nitrato-valeric acid (27.1 g, 0.166 mol) was dissolved in 1 L dry methylene chloride. PCl₅ (34.6 g, 0.166 mol) was added to the solution and the reaction mixture was stirred for 1.5 hours at ambient temperature. The reaction mixture was then concentrated and azeotroped with toluene to remove POCl₃. The acid chloride (29.7 g) was used without further purification.

### C. 5-Nitrato-valeric acid -[6-chloro-5-fluoro-2-oxindole-1-carboxamide-3-(2-thienylmethyl)] ester, (E) and (Z) isomer mixture

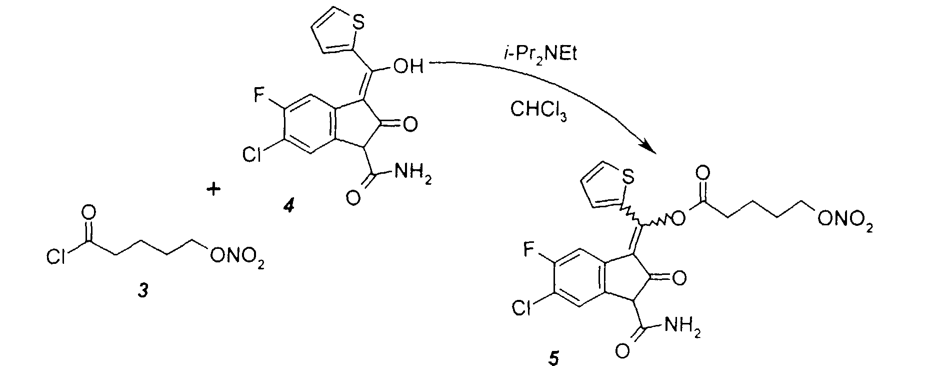

Acid chloride ***3*** (29.7 g, 0.164 mol, 1.42 equiv.) was dissolved in 800 mL distilled chloroform and added to a 2 liter flask containing 39.0 g ***4*** (0.115 mol). Diisopropylethylamine (28.4 mL, 0.163 mole, 1.42 equiv.) was dissolved in 200 mL of distilled chloroform and added via an addition funnel to the stirring reaction mixture. The deep orange solution was stirred for 1.5 hours. After diluting with chloroform 0.5 N HCI was added. A precipitate formed from unreacted ***4***. The organic solution was washed with water, dilute aqueous sodium carbonate, and then brine, and then finally evaporated to dryness. The crude product was chromatographed on 900 g silica gel, eluting with 50:50 EtOAc/hexanes affording 17.85 g (33%) of the title compound, 5, as about a 3:1 ratio of isomers.

### D. 5-Nitrato-valeric acid-[6-chloro-5-fluoro-2-oxindole-1-carboxamide-3-(2-thienylmethyl)] ester, (E) and (Z) separate isomers

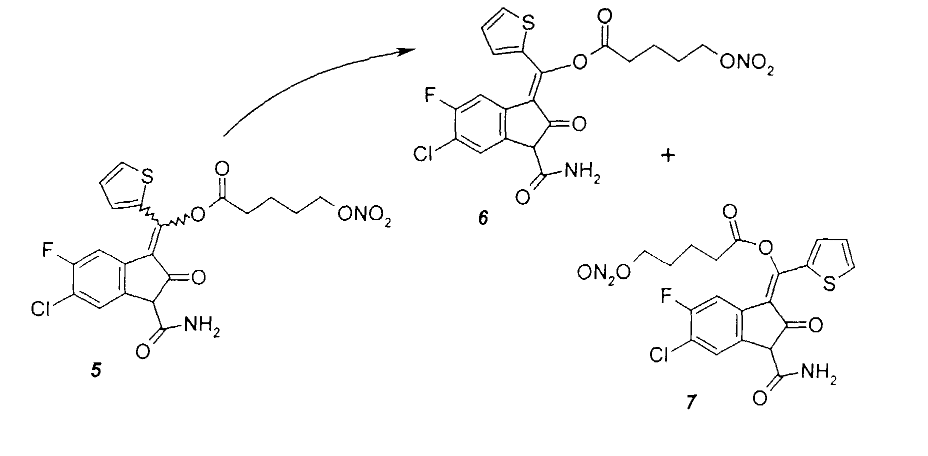

The 3:1 mixture of isomers was crystallized twice with hot acetonitrile to afford the pure Z isomer, ***6***, as a yellow solid (8.07 g, 15%, API MS = 484, mp 167-168%C, Anal. Calcd. for C₁₉H₁₅ClFN₃O₇S: C, 47.16, 11, 3.12; N. 8.68. Found C, 46.85; H, 3.09; N, 8.66). Three recrystallizations of the first mother liquor afforded pure E isomer, ***7***(1.34 g, API MS = 484, mp = 180-181°C, Anal. Calcd. for C₁₉H₁₅ClFN₃O₇S: C, 47.16; H, 3.12; N 8.68. Found: C, 47.14; H, 3.12; N, 8.70).

## Claims

1. A compound of Formula (I): 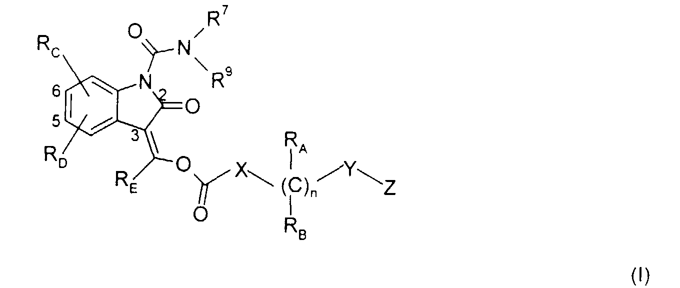 and pharmaceutically acceptable salts thereof; wherein
X is a covalent bond, -O-, -S-, or -N(R¹)- where R¹ is independently H or (C₁-C₄) alkyl wherein said alkyl may be straight or branched chain;
R_{A} and R_{B} are members independently selected from the group consisting essentially of H; (C₁-C₄) alkyl; (C₃-C₆) cycloalkyl, phenyl; or taken together with the carbon atom to which they are attached, form a bridging (C₃-C₆) cycloalkyl moiety; wherein said alkyl may be straight or branched chain, and wherein said alkyl, cycloalkyl, phenyl and bridging cycloalkyl moieties are independently substituted with 0 to 2 R³, where R³ is a member selected from the group consisting essentially of F, Cl, Br, CF₃, and (C₁-C₄) alkoxy;
n is an integer selected from 1 through 6, inclusive;
Y is a covalent bond, -O-, -S-, or -N-;
Z is -NO or -NO₂;
R_{C} is a member independently selected from the group consisting essentially of H, F, Cl, Br, (C₁-C₄) alkyl, (C₃-C₇) cycloalkyl, (C₁-C₄) alkoxy, (C₁-C₄) alkylthio, CF₃, (C₁-C₄) alkylsulfinyl, (C₁-C₄) alkylsulfonyl, NO₂, phenyl, (C₂-C₄) alkanoyl, benzoyl, thenoyl, (C₂ -C₄) alkanamido, benzamido, and N,N-di-(C₁-C₃) alkylsulfamoyl;
R_{D} is a member independently selected from the group consisting essentially of H, F, Cl, Br, (C₁-C₄) alkyl, (C₃-C₇) cycloalkyl, (C₁-C₄) alkoxy, (C₁-C₄) alkylthio, and CF₃; or
R_{C} and R_{D} when taken together are a 4,5-, 5,6- or 6,7-methylenedioxy group or a 4,5-, 5,6- or 6,7-ethylenedioxy group; or
R_{C} and R_{D} when taken together and when attached to adjacent carbon atoms, form a divalent radical D, wherein D is selected from the group consisting essentially of 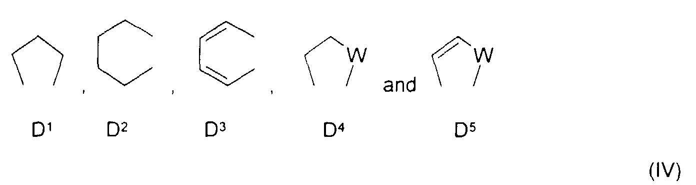
wherein W is O or S;
R_{E} is a member independently selected from the group consisting essentially of (C₁-C₆) alkyl, (C₃-C₇) cycloalkyl, (C₄-C₇) cycloalkenyl, phenyl, phenyl(C₁-C₃) alkyl, phenoxy(C₁-C₃) alkyl, thiophenoxy(C₁-C₃) alkyl, naphthyl, bicyclo[2.2.1]heptan-2-yl, bicyclo[2.2.1]hept-5-en-2-yl, and -(CH₂)ₘ-Q-R²; wherein said phenyl, phenylalkyl and phenoxyalkyl moieties are independently substituted with 0 to 2 R⁵, where R⁵ is a member selected from the group consisting essentially of F, Cl, Br, CF₃, (C₁-C₄)alkyl and (C₁-C₄) alkoxy; m is 0, 1 or 2; Q is a divalent radical derived from a compound independently selected from the group consisting essentially of furan, thiophene, pyrrole, pyrazole, imidazole, thiazole, isothiazole, oxazole, isoxazole, 1,2,3-thiadiazole, 1,3,4-thiadiazole, 1,2,5-thiadiazole, tetrahydrofuran, tetrahydrothiophene, tetrahydropyran, tetrahydrothiopyran, pyridine, pyrimidine, pyrazine, benzo[b]furan and benzo[b]thiophene; and R² is a member selected from the group consisting essentially of H, F, Cl, Br, CF₃, (C₁-C₃)alkyl, and (C₁-C₃) alkoxy; and
R⁷ and R⁹ are independently selected from the group consisting essentially of hydrogen, (C₁-C₃) alkyl, -C(=O) (C₁-C₃)alkyl, phenyl, and -C(=O)N(R⁸)(R¹⁰), where R⁸ and R¹⁰ are independently selected from the group consisting essentially of hydrogen, (C₁-C₃)alkyl, -C(=O)(C₁-C₃) alkyl, and phenyl.

2. A compound according to Claim 1 wherein X is a covalent bond; R_{A} and R_{B} are both hydrogen; n is the integer 4; Y is -O-; and Z is -NO₂.

3. A compound according to Claim 2 wherein R_{C} is a member selected from the group consisting essentially of 5-Cl and 5-F; R_{D} is a member selected from the group consisting essentially of 6-Cl and 6-F; and R_{E} is a member selected from the group consisting essentially of benzyl, 2-furyl, 2-thienyl, 5-chloro-2-thienyl and 5-trifluoromethyl-2-thienyl.

4. A compound according to Claim 1 wherein said compound is a member selected from the group consisting essentially of:
5-Nitrato-valeric acid-[6-chloro-5-fluoro-2-oxindole-1-carboxamide-3-(2-thienylmethyl)] ester, (*E*) and (*Z*) separate isomers;
5-S-nitroso-valeric acid-[6-chloro-5-fluoro-2-oxindole-1-carboxamide-3-(2-thienylmethyl)] ester;
3-S-nitroso-propylcarbamic acid-[6-chloro-5-fluoro-2-oxindole-1-carboxamide-3-(2-thienylmethyl)] ester;
2-(3,5-Difluorophenyl)-3-nitrato-propylcarbamic acid-[6-chloro-5-fluoro-2-oxindole-1-carboxamide-3-(2-thienylmethyl)] ester;
4-(3,5-Dimethoxyphenyl)-5-nitrato-valeric acid-[6-chloro-5-fluoro-2-oxindole-1-carboxamide-3-(2-thienylmethyl)] ester;
[6-Chloro-5-fluoro-2-oxindole-1-carboxamide-3-(2-thienylmethyl)oxy]formic acid 1-[4-(nitrato)]butyl ester;
[6-Chloro-5-fluoro-2-oxindole-1-carboxamide-3-(2-thienylmethyl)oxy]-S-thioformic acid 1-(2-cyclopropyl-3-nitrato)propyl ester;
[6-Chloro-5-fluoro-2-oxindole-1-carboxamide-3-(2-thienylmethyl)oxy]formic acid 1-[3-(1-*tert*-butyl-2-nitrato)ethyl]cyclopentyl ester;
[6-Chloro-5-fluoro-2-oxindole-1-carboxamide-3-(2-thienylmethyl)oxy]-S-thioformic acid (4-cyclohexyl4-nitrato-2-propyl)propyl ester;
[6-Chloro-5-fluoro-2-oxindole-1-carboxamide-3-(2-thienylmethyl)oxy]-S-thioformic acid 2-[(2-methoxy)phenyl-3-nitrato]propyl ester; and
[6-Chloro-5-fluoro-2-oxindole-1-carboxamide-3-(2-thienylmethyl)oxy]formic acid 2-[(3,6-difluorophenyl)-5-nitrato]pentyl ester.

5. A non-enteropathogenic method of treating osteoarthritis, an inflammatory disease or condition, or a degenerative joint disease or condition in a patient in need of such treatment, comprising administering to said patient a therapeutically effective but non-enteropathogenic amount of a compound of Formula (I) as defined in Claim 1.

6. A non-enteropathogenic method of treating and modifying an osteoarthritic, inflammatory, or degenerative joint disease or condition in a pateint in need of such treatment, comprising administering to said patient a non-enteropathogenic amount of a compound of Formula (I), as defined in claim 1, which is therapeutically effective in halting or reversing the early stages of degeneration and decline in said patient's body tissues, organs, and basic functions associated with said disease or condition, whereby the outcome of said disease or condition in said patient is favorably affected.

7. A non-enteropathogenic pharmaceutical composition for treating osteoarthritis, an inflammatory disease or condition, or a degenerative joint disease or condition in a patient in need of such treatment, comprising a pharmaceutically acceptable carrier and a compound of Formula (I) as defined in Claim 1.

8. A non-enteropathogenic pharmaceutical compositions for use in modifying the course of an osteoarthritic, inflammatory, or degenerative joint disease or condition in a patient in need of such treatment, comprising a non-enteropathogenic amount of a compound of Formula (I), as defined in claim 1, which is therapeutically effective in halting or reversing the early stages of degeneration and decline in said patient's body tissues, organs, and basic functions associated with said disease or condition, together with a pharmaceutically acceptable carrier for said compound of Formula (I).

9. A pharmaceutical composition comprising a compound of Formula (I) as defined in Claim 1 in combination with one or more other therapeutically active agents under the following conditions:
A. where a joint has become seriously inflammed as well as infected at the same time by bacteria, fungi, protozoa, and/or virus, said inhibitory compound is administered in combination with one or more antibiotic, antifungal, antiprotozoal, and/or antiviral therapeutic agents; or
B. where a multi-fold treatment of pain and inflammation is desired, said inhibitory compound is administered in combination with inhibitors of other mediators of inflammation, comprising one or more members independently selected from the group consisting essentially of:
1. NSAIDs;
2. H₁-receptor antagonists;
3. kinin-B₁ - and B₂ -receptor antagonists;
4. prostaglandin inhibitors selected from the group consisting of PGD-, PGF- PGI₂ -, and PGE-receptor antagonists;
5. thromboxane A₂ (TXA2-) inhibitors;
6. 5- and 12-lipoxygenase inhibitors;
7. leukotriene LTC₄ -, LTD₄/LTE₄ -, and LTB₄ -inhibitors;
8. PAF-receptor antagonists;
9. gold in the form of an aurothio group together with one or more hydrophilic groups;
10. immunosuppressive agents selected from the group consisting of cyclosporine, azathioprine, and methotrexate;
11. anti-inflammatory glucocorticoids;
12. penicillamine;
13. hydroxychloroquine;
14. anti-gout agents including colchicine; xanthine oxidase inhibitors including allopurinol; and uricosuric agents selected from probenecid, sulfinpyrazone, and benzbromarone.
